# EUROPEAN PATENT APPLICATION

(11) **EP 2 591 775 A1**
(43) Date of publication of application: **15.05.2013**
(21) Application number: 13152306.0
(22) Date of filing: 05.04.2007
(51) Int. Cl.: A61K 31/132, A61K 31/136, A61K 31/282, A61K 31/436, A61K 31/437, A61K 31/475, A61K 31/5375, A61K 31/573, A61K 31/675, A61K 31/704, A61K 31/7068, A61K 31/7076, A61K 45/06, A61P 35/00, A61P 35/02, A61P 35/04

(54) **Combinations comprising mtor inhibitors for treating cancer**

(30) Priority: 05.04.2006 US 789400 P
(62) Divisional of application: 07781370.7
(71) Applicant: Novartis AG, 4056 Basel (CH); Novartis Pharma AG, 1230 Vienna (AT)
(72) Inventor: Burke, Gregory, Randolph, NJ New Jersey 07869 (US); Linnartz, Ronald Richard, Andover, NJ New Jersey 07821 (US); Versage, Richard William, Wanaque, NJ New Jersey 07465 (US); Lane, Heidi, 4105 Biel-Benken (CH)
(74) Representative: Gruber, Markus

(57) **Abstract**

A combination of an mTOR inhibitor compound with one or more pharmaceutically active agents, for simultaneous, concurrent, separate or sequential use for preventing or treating a proliferative disease.

## Description

The present invention relates to a combination comprising an mTOR inhibitor; and one or more pharmaceutically active agents; pharmaceutical compositions comprising said combination; methods of treatment comprising said combination; processes for making said combination; and a commercial package comprising said combination.

### Background of the Invention

Rapamycin and rapamycin derivatives are inhibitors of mTOR activity and exhibit a wide array of biological activities. It is also known that different combinations of active ingredients may increase anti-tumor behaviour. Therefore, there is a continuing need for new combinations of rapamycin and rapamycin derivatives, especially 40-O-(2-hydroxyethyl)rapamycin.

### Summary of the Invention

In one aspect the present invention provides a combination which comprises:
(a) a mTOR inhibitor; and
(b) one or more pharmaceutically active agents.

In another aspect he present invention further provides a pharmaceutical compositions comprising:
(a) a mTOR inhibitor;
(b) a pharmaceutically active agent; and
(c) a pharmaceutically acceptable carrier.

In another aspect the present invention further provides a commercial package or product comprising:
(a) a pharmaceutical formulation of a mTOR inhibitor; and
(b) a pharmaceutical formulation of a pharmaceutically active agent for simultaneous, concurrent, separate or sequential use.

The combination partners (a) and (b) can be administered together, one after the other or separately in one combined unit dosage form or in two separate unti dosage forms. The unit dosage form may also be a fixed combination.

Combinations according to the present invention include fixed combinations, in which a mTOR inhibitor compound and one or more pharmaceutically active agents are in the same formulation; kits, in which a mTOR inhibitor compound and one or more pharmaceutically active agents in separate formulations are provided in the same package, e.g. with instruction for co-administration; and free combinations in which mTOR inhibitor compound and one or more pharmaceutically active agents are packaged separately, but instruction for concomitant or sequential administration are given.

In another aspect the present invention provides
- A pharmaceutical package comprising a mTOR inhibitor compound and and one or more pharmaceutically active agents, beside instructions for combined administration;
- A pharmaceutical package comprising a mTOR inhibitor compound beside instructions for combined administration with one or more pharmaceutically active agents;
- A pharmaceutical package comprising one or more pharmaceutically active agents beside instructions for combined administration with a mTOR inhibitor compound;
which is a commercial package or product.

In another aspect the present invention provides a method of preventing or treating proliferative diseases or diseases that are associated with or triggered by persistent angiogenesis in a mammal, particularly a human, with a combination comprising:
(a) a mTOR inhibitor; and
(b) one or more pharmaceutically active agents.

In another aspect the present invention provides the use of a combination, pharmaceutical composition or commercial package provided by the present invention for the manufacture of a medication for preventing or treating proliferative diseases or diseases that are associated with or triggered by persistent angiogenesis in a mammal.

### Brief Description of the Drawings

**FIG 1:** shows the percent inhibition for a 81-point 9x9 dose matrix for the combination with 40-O-(2-hydroxyethyl)rapamycin and vinorelbine in A549 cells
**FIG 2:** shows the synergy for each dose point compared to the Loewe additivity model for the combination with 40-O-(2-hydroxyethyl)rapamycin and vinorelbine in A549 cells
**FIG 3:** shows the isobologram contour at 30% inhibition for the combination with 40-O-(2-hydroxyethyl)rapamycin and vinorelbine in A549 cells
**FIG 4:** shows percent inhibition for a 81-point 9x9 dose matrix for the combination with 40-O-(2-hydroxyethyl)rapamycin and 8-phenyl-2-(morpholin-4-yl)-chromen-4-one in A549 cells.
**FIG 5:** shows the synergy for each dose point compared to the Loewe additivity model for the combination with 40-O-(2-hydroxyethyl)rapamycin and 8-phenyl-2-(morpholin-4-yl)-chromen-4-one in A549 cells.
**FIG 6:** .shows the the isobologram contour at 25% inhibition for the combination with 40-O-(2-hydroxyethyl)rapamycin and 8-phenyl-2-(morpholin-4-yl)-chromen-4-one in A549 cells.

### Detailed Description of the Invention

### I. The mTOR Inhibitor

### DETAILED DESCRIPTION OF THE INHIBITOR

The phrase "mTOR inhibitor" as used herein, includes but is not limited to compounds, proteins or antibodies which target/inhibit the activity of members of the mTOR kinase family. Inhibitors of mTOR activity e.g. include rapamycin of formula 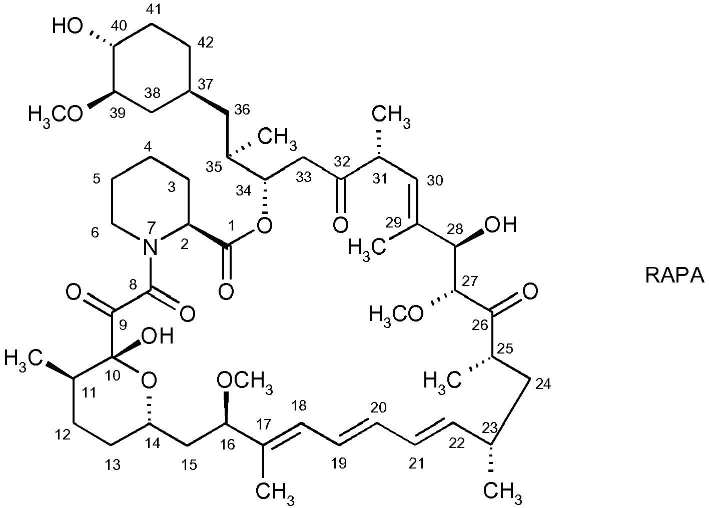 and rapamycin derivatives, e.g. including
40-O-substituted rapamycin derivatives, such as
40-O-alkyl-rapamycin derivatives, such as 40-O-hydroxyalkyl-rapamycin derivatives, such as 40-O-(2-hydroxy)-ethyl-rapamycin (everolimus),
32-deoxo-rapamycin derivatives and 32-hydroxy-rapamycin derivatives, such as 32-deoxorapamycin,
16-O-substituted rapamycin derivatives such as 16-pent-2-ynyloxy-32-deoxorapamycin, 16-pent-2-ynyloxy-32(S or R) -dihydro-rapamycin, 16-pent-2-ynyloxy-32(S or R)-dihydro-40-O-(2-hydroxyethyl)-rapamycin,
rapamycin derivatives which are acylated at the oxygen group in position 40, e.g. 40-[3-hydroxy-2-(hydroxy-methyl)-2-methylpropanoate]-rapamycin (also known as CCl779), rapamycin derivatives which are substituted in 40 position by heterocyclyl, e.g. 40-epi-(tetrazolyl)-rapamycin (also known as ABT578),
the so-called rapalogs, e. g. as disclosed in WO9802441 or WO0114387, e.g. such as 40-O-phospho-containing rapamycin derivatives, e.g. 40-O-dimethylphosphinyl-rapamycin, including AP23573, and
40-O-alkoxy-alkyl-rapamycin derivatives, such as compounds as disclosed under the name biolimus (biolimus A9), including 40-O-(2-ethoxy)-ethyl-rapamycin, and compounds disclosed under the name TAFA-93, AP23464, AP23675 or AP23841;
preferably 40-O-(2-hydroxy)-ethyl-rapamycin, CCI779, ABT578, or AP23573, more preferably 40-O-(2-hydroxy)-ethyl-rapamycin (everolimus).

In one embodiment of the present invention a preferred group of rapamycin derivatives includes a compound of formula 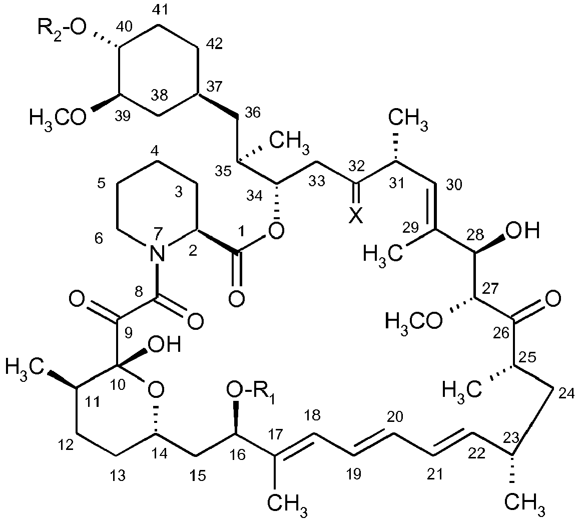 wherein
R₁ is CH₃ or C₃₋₆alkynyl,
R₂ is H or -CH₂-CH₂-OH, and
X is =O, (H,H) or (H,OH)
provided that R₂ is other than H when X is =O and R₁ is CH₃.

Preferred compounds are 32-deoxorapamycin, 16-pent-2-ynyloxy-32-deoxorapamycin, 16-pent-2-ynyloxy-32(S)-dihydro-rapamycin, 16-pent-2-ynyloxy-32(S)-dihydro-40-O-(2-hydroxyethyl)-rapamycin and, more preferably, 40-O-(2-hydroxy)-ethyl-rapamycin.

Compounds of formula I have, on the basis of observed activity, e.g. binding to macrophilin-12 (also known as FK-506 binding protein or FKBP-12), been found to be useful as immunosuppressant, e.g. in the treatment of acute allograft rejection and as having potent antiproliferative properties which make them useful for cancer chemotherapy, particularly of solid tumors, especially of advanced solid tumors.

### II. The Pharmaceutically Active Agents

The term " pharmaceutically active agents" is a broad one covering many pharmaceutically active agents having different mechanisms of action. Combinations of some of these with mTOR inhibitors can result in improvements in cancer therapy. Generally, pharmaceutically active agents are classified according to the mechanism of action. Many of the available agents are anti-metabolites of development pathways of various tumors, or react with the DNA of the tumor cells. There are also agents which inhibit enzymes, such as topoisomerase I and topoisomerase II, or which are antimiotic agents.

By the term " pharmaceutically active agent" is meant especially any pharmaceutically active agent other than a mTOR inhibitor or a derivative thereof. It includes, but is not limited to:
i. a steroid;
ii. an adenosine-kinase-inhibitor;
iii. an adjuvant;
iv. an adrenal cortex antagonist;
v. AKT pathway inhibitor;
vi. An alkylating agent;
vii. an angiogenesis inhibitor;
viii. an anti-androgen;
ix. an anti-estrogen;
x. an anti-hypercalcemia agent;
xi. an antimetabolite;
xii. an apoptosis inducer;
xiii. an aurora kinase inhibitor;
xiv. a Bruton's Tyrosine Kinase (BTK) inhibitor;
xv. a calcineurin inhibitor;
xvi. a CaM kinase II inhibitor;
xvii. a CD45 tyrosine phosphatase inhibitor;
xviii. a CDC25 phosphatase inhibitor;
xix. a CHK kinase inhibitor;
xx. a controlling agent for regulating genistein, olomucine and/or tyrphostins;
xxi. a cyclooxygenase inhibitor;
xxii. a cRAF kinase inhibitor;
xxiii. a cyclin dependent kinase inhibitor;
xxiv. a cysteine protease inhibitor;
xxv. a DNA intercalator;
xxvi. a DNA strand breaker;
xxvii. an E3 Ligase inhibitor;
xxviii. an endocrine hormone;
xxix. compounds targeting, decreasing or inhibiting the activity of the epidermal growth factor family;
xxx. an EGFR, PDGFR tyrosine kinase inhibitor;
xxxi. a farnesyltransferase inhibitor;
xxxii. a Flk-1 kinase inhibitor;
xxxiii. a Glycogen synthase kinase-3 (GSK3) inhibitor;
xxxiv. a histone deacetylase (HDAC) inhibitor;
xxxv. a HSP90 inhibitor;
xxxvi. a I-kappa B-alpha kinase inhibitor (IKK);
xxxvii. an insulin receptor tyrosine kinase inhibitor;
xxxviii. a c-Jun N-terminal kinase (JNK) kinase inhibitor;
xxxix. a microtubule binding agent;
xl. a Mitogen-activated protein (MAP) kinase-inhibitor;
xli. a MDM2 inhibitor;
xlii. a MEK inhibitor;
xliii. a matrix metalloproteinase inhibitor (MMP) inhibitor;
xliv. a NGFR tyrosine-kinase-inhibitor;
xlv. a p38 MAP kinase inhibitor, including a SAPK2/p38 kinase inhibitor;
xlvi. a p56 tyrosine kinase inhibitor;
xlvii. a PDGFR tyrosine kinase inhibitor;
xlviii. a phosphatidylinositol 3-kinase inhibitor;
xlix. a phosphatase inhibitor;
I. a platinum agent;
li. a protein phosphatase inhibitor, including a PP1 and PP2 inhibitor and a tyrosine phosphatase inhibitor;
lii. a PKC inhibitor and a PKC delta kinase inhibitor;
liii. a polyamine synthesis inhibitor;
liv. a proteosome inhibitor;
lv. a PTP1B inhibitor;
lvi. a protein tyrosine kinase inhibitor including a SRC family tyrosine kinase inhibitor; a Syk tyrosine kinase inhibitor; and a JAK-2 and/or JAK-3 tyrosine kinase inhibitor;
lvii. a retinoid;
lviii. a RNA polymerase II elongation inhibitor;
lix. a serine/threonine kinase inhibitor;
lx. a sterol biosynthesis inhibitor;
lxi. a topoisomerase inhibitor;
lxii. VEGFR tyrosine kinase inhibitor,
lxiii. a gonadorelin agonist,
lxiv. a compound which induce cell differentiation processes,
lxv. a bisphosphonate,
lxvi. a heparanase inhibitor,
lxvii. a biological response modifier,
lxviii. a telomerase inhibitor,
lxix. mediators, such as inhibitors of catechol-O-methyltransferase,
lxx: an immunosuppressive monoclonal antibody, e.g. monoclonal, to leukocyte receptors,
lxxi somatostatin or a somatostatin analogue,
lxxii. Growth Hormone-Receptor Antagonists,:
lxxiii. monoclonal antibodies useful for leukemia (AML) treatment,
lxxiv. antibodies against carcinoembryonic antigen (CEA),
lxxv. a phosphodiesterase inhibitor,
lxxvi. a cancer vaccine,
lxxvii. inhibitors of Kinesin Spindle Protein (KSP),
lxxviii. inhibitors of multiple receptor tyrosine kinases associated with tumour growth and angiogenesis,
lxxix. synthetic nonsteroidal estrogens,
lxxx. cytoxic antineoplastics,
lxxxi. a recombinant binding molecule having at least a portion of the extracellular domain of CTLA4 or a mutant thereof.

The term "a steroid", as used herein, relates to Prednisone.

The term "an adenosine-kinase-inhibitor", as used herein, relates to a compound which targets, decreases or inhibits nucleobase, nucleoside, nucleotide and nucleic acid metabolisms. An example of an adenosine-kinase-inhibitor includes, but is not limited to, 5-lodotubercidin, which is also known as 7H-pyrrolo[2,3-d]pyrimidin-4-amine, 5-iodo-7-β-D-ribofuranosyl.

The term "an adjuvant", as used herein, refers to a compound which enhances the 5-FU-TS bond as well as a compound which targets, decreases or inhibits, alkaline phosphatase. Examples of an adjuvant include, but are not limited to, Leucovorin, and Levamisole.

The term "an adrenal cortex antagonist", as used herein, relates to a compound which targets, decreases or inhibits the activity of the adrenal cortex and changes the peripheral metabolism of corticosteroids, resulting in a decrease in 17-hydroxycorticosteroids. An example of an adrenal cortex antagonist includes, but is not limited to, Mitotane.

The term "AKT pathway inhibitor", as used herein, relates to a compound which targets, decreases or inhibits cell proliferation. Akt, also known as protein kinase B (PKB), a serine/threonine kinase, is a critical enzyme in several signal transduction pathways involved in diabetes. The principal role of Akt in the cell is to facilitate growth factor-mediated cell survival and to block apoptotic cell death. A target of the AKT pathway inhibitor includes, but is not limited to, Pi3K/AKT. Examples of an AKT pathway inhibitor, include, but are not limited to, Deguelin, which is also known as 3H-bis[1]benzopyrano[3,4-b:6',5'-e]pyran-7(7aH)-one, 13,13a-dihydro-9,10-dimethoxy-3,3-dimethyl-, (7aS, 13aS); Triciribine, which is also known as 1,4,5,6,8-pentaazaacenaphthylen-3-amine, 1,5-dihydro-5-methyl-1-β-D-ribofuranosyl and KP372-1 (QLT394).

The term "an alkylating agent", as used herein, relates to a compound which causes alkylation of DNA and results in breaks in the DNA molecules as well as cross-linking of the twin strands, thus interfering with DNA replication and transcription of RNA. Examples of an alkylating agent include, but are not limited to, such as chlorambucil, chlormethine, cyclophosphamide, ifosfamide, melphalan, estramustine; nitrosueras, such as carmustine, fotemustine, lomustine, streptozocin (streptozotocin, STZ), BCNU; Gliadel; dacarbazine, mechlorethamine, e.g. in the form of a hydrochloride, procarbazine, e.g. in the form of a hydrochloride, thiotepa, temozolomide, nitrogen mustard, mitomycin, altretamine, busulfan, estramustine, uramustine. Cyclophosphamide can be administered, e.g., in the form as it is marketed, e.g., under the trademark CYCLOSTIN®; ifosfamide as HOLOXAN®, temozolomide as TEMODAR®, nitrogen mustard as MUSTARGEN®, estramustine as EMYCT®, streptozocin as ZANOSAR®.

The term "an angiogenesis inhibitor", as used herein, relates to a compound which targets, decreases or inhibits the production of new blood vessels. Targets of an angiogenesis inhibitor include, but are not limited to, methionine aminopeptidase-2 (MetAP-2), macrophage inflammatory protein-1 (MIP-1alpha), CCL5, TGF-beta, lipoxygenase, cyclooxygenase, and topoisomerase. Indirect targets of an angiogenesis inhibitor include, but are not limited to, p21, p53, CDK2, and collagen synthesis. Examples of an angiogenesis inhibitor include, but are not limited to, Fumagillin, whichis known as 2,4,6,8-Decatetraenedioic acid, mono[(3R,4S,5S,6R)-5-methoxy-4-[(2R,3R)-2-methyl-3-(3-methyl-2-butenyl)oxiranyl]-1-oxaspiro[2.5]oct-6-yl] ester, (2E,4E,6E,8E)-; Shikonin, which is also known as 1,4-Naphthalenedione, 5,8-dihydroxy-2-[(1R)-1-hydroxy-4-methyl-3-pentenyl]; Tranilast, which is also known as benzoic acid, 2-[[3-(3,4-dimethoxyphenyl)-1-oxo-2-propenyl]amino]-(9Cl); ursolic acid; suramin; bengamide or a derivative thereof, TNP-470.and thalidomide.

The term "an anti-androgen", as used herein, relates to a compound which blocks the action of androgens of adrenal and testicular origin which stimulate the growth of normal and malignant prostatic tissue. Examples of an anti-androgen include, but are not limited to, Nilutamide; bicalutamide (CASODEX), which can be formulated, e.g., as disclosed in.US4636505.

The term "an anti-estrogen", as used herein, relates to a compound which antagonizes the effect of estrogens at the estrogen receptor level. Examples of an anti-estrogen include, but are not limited to, e.g. including atamestane, exemestane, formestane, aminoglutethimide, roglethimide, pyridoglutethimide, trilostane, testolactone, ketokonazole, vorozole, fadrozole, anastrozole, letrozole, toremifene; bicalutamide; flutamide; tamoxifen, tamoxifen citrate; tamoxifen; fulvestrant; raloxifene, raloxifene hydrochloride. Tamoxifen may be e.g. administered in the form as it is marketed, e.g., NOLVADEX®; and raloxifene hydrochloride is marketed as EVISTA®. Fulvestrant may be formulated as disclosed in US4659516 and is marketed as FASLODEX®. A combination of the invention comprising a pharmaceutically active agent which is an anti-estrogen is particularly useful for the treatment of estrogen receptor positive tumors, e.g., breast tumors.

The term "an anti-hypercalcemia agent", as used herein, refers to compounds which are used to treat hypercalcemia. Examples of an anti-hypercalcemia agent include, but are not limited to, gallium (III) nitrate hydrate; and pamidronate disodium.

The term "antimetabolite", as used herein, relates to a compound which inhibits or disrupts the synthesis of DNA resulting in cell death. Examples of an antimetabolite include, but are not limited to, DNA de-methylating agents and folic acid antagonists, e.g. methotrexate, pemetrexed (permetrexed, Alimta®, raltitrexed; purins, e.g. 6-mercaptopurine, cladribine, clofarabine; fludarabine, thioguanine (tioguanine), 6-thioguanine, nelarabine (compound 506), tiazofurin (inhibits inosine monophosphate dehydrogenase and guanosine triphosphate pools), pentostatin (deoxycoformycin); cytarabine; flexuridine; fluorouracil; 5-fluorouracil (5-FU), floxuridine (5-FUdR), capecitabine; gemcitabine; gemcitabine hydrochloride; hydroxyurea (e.g. Hydrea®); DNA de-methylating agents, such as 5-azacytidine (Vidaza®) and decitabine; fluoromethylene deoxycitidine (FmdC), 5-aza-2'-deoxycytidine, troxacitabine (L-isomer cytosine analogue), edatrexate;. Capecitabine and gemcitabine can be administered e.g. in the marketed form, such as XELODA® and GEMZAR®.

The term "an apoptosis inducer", as used herein, relates to a compound which induces the normal series of events in a cell that leads to its death. The apoptosis inducer of the present invention may selectively induce the X-linked mammalian inhibitor of apoptosis protein XIAP. The apoptosis inducer of the present invention may downregulate BCL-xL. Examples of an apoptosis inducer include, but are not limited to, ethanol, 2-[[3-(2,3-dichlorophenoxy)propyl]amino]-(9Cl); gambogic acid; Embelin, which is also known as 2,5-Cyclohexadiene-1,4-dione, 2,5-dihydroxy-3-undecyl; and Arsenic Trioxide (TRISENOX®).

The term "an aurora kinase inhibitor", as used herein, relates to a compound which targets, decreases or inhibits later stages of the cell cycle from the G2/M check point all the way through to the mitotic checkpoint and late mitosis. An example of an aurora kinase inhibitor includes, but is not limited to Binucleine 2, which is also known as Methanimidamide, N'-[1-(3-chloro-4-fluorophenyl)-4-cyano-1H-pyrazol-5-yl]-N,N-dimethyl.

The term "a Bruton's Tyrosine Kinase (BTK) inhibitor", as used herein, relates to a compound which targets, decreases or inhibits human and murine B cell development. An example of a BTK inhibitor includes, but is not limited to terreic acid.

The term "a calcineurin inhibitor", as used herein, relates to a compound which targets, decreases or inhibits the T cell activation pathway. A target of a calcineurin inhibitor includes protein phosphatase 2B. Examples of a calcineurin inhibitor include, but are not limited to Cypermethrin, which is also known as cyclopropanecarboxylic acid, 3-(2,2-dichloroethenyl)-2,2-dimethyl-,cyano(3-phenoxyphenyl)methyl ester; Deltamethrin, which is also known as cyclopropanecarboxylic aci, 3-(2,2-dibromoethenyl)-2,2-dimethyl-(S)-cyano(3-phenoxyphenyl)methyl ester, (1 R,3R); Fenvalerate, which is also known as benzeneacetic acid, 4-chloro-α-(1-methylethyl)-,cyano(3-phenoxyphenyl)methyl ester; and Tyrphostin 8.

The term "a CaM kinase II inhibitor", as used herein, relates to a compound which targets, decreases or inhibits CaM Kinases. CaM Kinases constitute a family of structurally related enzymes that include phosphorylase kinase, myosin light chain kinase, and CaM kinases I-IV. CaM Kinase II, one of the best-studied multifunctional enzymes, is found in high concentrations in neuronal synapses, and in some regions of the brain it may constitute up to 2% of the total protein content. Activation of CaM kinase II has been linked to memory and learning processes in the vertebrate nervous system. Targets of a CaM kinase II inhibitor include CaM kinase II. Examples of a CaM kinase II inhibitor include, but are not limited to, 5-Isoquinolinesulfonic acid, 4-[(2S)-2-[(5-isoquinolinylsulfonyl)methylamino]-3-oxo-3-(4-phenyl-1-piperazinyl)propyl]phenyl ester; and benzenesulfonamide, N-[2-[[[3-(4-chlorophenyl)-2-propenyl]methyl]amino]methyl]phenyl]-N-(2-hydroxyethyl)-4-methoxy-.

The term "a CD45 tyrosine phosphatase inhibitor", as used herein, relates to a compound which targets, decreases or inhibits dephosphorylating regulatory pTyr residues on Src-family protein-tyrosine kinases, which aids in the treatment of a variety of inflammatory and immune disorders. An example of a CD45 tyrosine phosphatase inhibitor includes, but is not limited to, Phosphonic acid, [[2-(4-bromophenoxy)-5-nitrophenyl]hydroxymethyl]-.

The term "a CDC25 phosphatase inhibitor", as used herein, relates to compound which targets, decreases or inhibits overexpressed dephosphorylate cyclin-dependent kinases in tumors. An example of a CDC25 phosphatase inhibitor includes 1,4-naphthalenedione, 2,3-bis[(2-hydroyethyl)thio]-.

The term "a CHK kinase inhibitor", as used herein, relates to a compound which targets, decreases or inhibits overexpression of the antiapoptotic protein Bcl-2. Targets of a CHK kinase inhibitor are CHK1 and/or CHK2. An example of a CHK kinase inhibitor includes, but is not limited to, Debromohymenialdisine.

Examples of a "controlling agent for regulating genistein, olomucine and/or tyrphostins" includes, but are not limited to, Daidzein, which is also known as 4H-1-benzopyran-4-one, 7-hydroxy-3-(4-hydroxyphenyl)-(9Cl); Iso-Olomoucine, and Tyrphostin 1.

The term "cyclooxygenase inhibitor" as used herein includes, but is not limited to, e.g., Cox-2 inhibitors. The term "a COX-2 inhibitor", as used herein, relates to a compound which targets, decreases or inhibits the enzyme cox-2 (cyclooxygenase-2). Examples of a COX-2 inhibitor, include but are not limited to, 1 H-indole-3-acetamide, 1-(4-chlorobenzoyl)-5-methoxy-2-methyl-N-(2-phenylethyl)-; 5-alkyl substituted 2-arylaminophenylacetic acid and derivatives, such as celecoxib (CELEBREX), rofecoxib (VIOXX®), etoricoxib, valdecoxib; or a 5-alkyl-2-arylaminophenylacetic acid, e.g., 5-methyl-2-(2'-chloro-6'-fluoroanilino)phenyl acetic acid, lumiracoxib; and celecoxib.

The term "a cRAF kinase inhibitor", as used herein, relates to a compound which targets, decreases or inhibits the up-regulation of E-selectin and vascular adhesion molecule-1 induced by TNF. Raf kinases play an important role as extracellular signal-regulating kinases in cell differentiation, proliferation, and apoptosis. A target of a cRAF kinase inhibitor includes, but is not limited, to RAF1. Examples of a cRAF kinase inhibitor include, but are not limited to, 3-(3,5-dibromo-4-hydroxybenzylidene)-5-iodo-1,3-dihydroindol-2-one; and benzamide, 3-(dimethylamino)-N-[3-[(4-hydroxybenzoyl)amino]-4-methylphenyl]. RAF kinase inhibitors e.g. include compounds as described in WO2005028444 or WO0009495.

The term "a cyclin dependent kinase inhibitor", as used herein, relates to a compound which targets, decreases or inhibits cyclin dependent kinase which play a role in the regulation of the mammalian cell cycle. Cell cycle progression is regulated by a series of sequential events that include the activation and subsequent inactivation of cyclin dependent kinases (Cdks) and cyclins. Cdks are a group of serine/threonine kinases that form active heterodimeric complexes by binding to their regulatory subunits, cyclins. Examples of targets of a cyclin dependent kinase inhibitor include, but are not limited to, CDK, AHR, CDK1, CDK2, CDK5, CDK4/6, GSK3beta, and ERK. Examples of a cyclin dependent kinase inhibitor include, but are not limited to, N9-Isopropyl-Olomoucine; Olomoucine; Purvalanol B, which is also known as Benzoic acid, 2-chloro-4-[[2-[[(1R)-1-(hydroxymethyl)-2-methylpropyl]amino]-9-(1-methylethyl)-9H-purin-6-yl]amino]-; Roascovitine; Indirubin, which is also known as 2H-Indol-2-one, 3-(1,3-dihydro-3-oxo-2H-indol-2-ylidene)-1,3-dihydro-; Kenpaullone, which is also known as Indolo[3,2-d][1]benzazepin-6(5H)-one, 9-bromo-7,12-dihydro-; purvalanol A, which is also known as 1-Butanol, 2-[[6-[(3-chlorophenyl)amino]-9-(1-methylethyl)-9H-purin-2-yl]amino]-3-methyl-, (2R)-, and Indirubin-3'-monooxime.

The term "a cysteine protease inhibitor", as used herein, relates to a compound which targets, decreases or inhibits cystein protease which plays a vital role in mammalian cellular turnover and apotosis. An example of a cystein protease inhibitor includes, but is not limited to, 4-morpholinecarboxamide,N-[(1S)-3-fluoro-2-oxo-1-(2-phenylethyl)propyl]amino]-2-oxo-1-(phenylmethyl)ethyl]-.

The term "a DNA intercalator" as used herein, relates to a compound which binds to DNA and inhibits DNA, RNA, and protein synthesis. Examples of a DNA intercalator include, but are not limited to, Plicamycin and Dactinomycin.

The term "a DNA strand breaker" as used herein, relates to a compound which causes DNA strand scission and results in inhibition of DNA synthesis, ininhibition of RNA and protein synthesis. An example of a DNA strand breaker includes, but is not limited to, Bleomycin.

The term "an E3 Ligase inhibitor", as used herein, relates to a compound which targets, decreases or inhibits the E3 ligase which inhibits the transfer of ubiquitin chains to proteins, marking them for degradation in the proteasome. An example of a E3 ligase inhibitor includes, but is not limited to, N-((3,3,3-trifluoro-2-trifluoromethyl)propionyl)sulfa nilamide.

The term "an endocrine hormone", as used herein, relates to a compound which by acting mainly on the pituitary gland causes the suppression of hormones in males, the net effect is a reduction of testosterone to castration levels. In females, both ovarian estrogen and androgen synthesis are inhibited. An example of an endocrine hormone includes, but is not limited to, Leuprolide and megestrol and megestrol acetate.

The term "compounds targeting, decreasing or inhibiting the activity of the epidermal growth factor family", as used herein, relates to a compound which icompounds targeting, decreasing or inhibiting the activity of the epidermal growth factor family of receptor tyrosine kinases (EGFR, ErbB2, ErbB3, ErbB4 as homo- or heterodimers), such as compounds which target, decrease or inhibit the activity of the epidermal growth factor receptor family are especially compounds, proteins or antibodies which inhibit members of the EGF receptor tyrosine kinase family, e.g., EGF receptor, ErbB2, ErbB3 and ErbB4 or bind to EGF or EGF-related ligands, and are in particular those compounds, proteins or monoclonal antibodies generically and specifically disclosed in WO9702266, e.g., the compounds in EP0564409, WO9903854, EP0520722, EP0566226, EP0787722, EP0837063, US5747498, WO9810767, WO 97/30034, WO9749688, WO9738983 and, especially, WO9630347, e.g., a compound known as CP 358774, WO9633980, e.g., compound ZD 1839; and WO9503283, e.g., compound ZM105180, e.g including the dual acting tyrosine kinase inhibitor (ErbB1 and ErbB2) lapatinib (GSK572016), e.g. lapatinib ditosylate; panituzumab, trastuzumab (HERCEPTIN^{®}), cetuximab, Iressa, OSI-774, Cl-1033, EKB-569, GW-2016, E1.1, E2.4, E2.5, E6.2, E6.4, E2.11, E6.3 or E7.6.3, and 7H-pyrrolo-[2,3-d]pyrimidine derivatives which are disclosed in WO03013541, vatanalib,erlotinib and gefitinib. Erlotinib can be administered in the form as it is marketed, e.g. TARCEVA®, and gefitinib as IRESSA®, human monoclonal antibodies against the epidermal growth factor receptor including ABX-EGFR. Targets of an EGFR kinase inhibitor include, but are not limited to, guanylyl cyclase (GC-C) and HER2. Other examples of an EGFR kinase inhibitor include, but are not limited to, zalutumumab, Tyrphostin 23, Tyrphostin 25, Tyrphostin 47, Tyrphostin 51 and Tyrphostin AG 825. Targets of an EGFR tyrosine kinase inhibitor include EGFR, PTK and tubulin. Other examples of an EGFR tyrosine kinase inhibitor include, but are not limited to, 2-propenamide, 2-cyano-3-(3,4-dihydroxyphenyl)-N-phenyl-,(2E)-; Tyrphostin Ag 1478; Lavendustin A; and 3-pyridineacetonitrile, α-[(3,5-dichlorophenyl)methylene]-, (αZ)-(9Cl). An example of an EGFR, PDGFR tyrosine kinase inhibitor includes, but is not limited to, Tyrphostin 46.

The term "a farnesyltransferase inhibitor", as used herein, relates to a compound which targets, decreases or inhibits the Ras protein, which is commonly abnormally active in cancer. A target of a farnesyltransferase inhibitor includes, but is not limited to RAS. Examples of a farnesyltransferase inhibitor include, but are not limited to a hydroxyfarnesylphosphonic acid; butanoic acid, 2-[[(2S)-2-[[(2S,3S)-2-[[(2R)-2-amino-3-mercaptopropyl]amino]-3-methylpentyl]oxy]-1-oxo-3-phenylpropyl]amino]-4-(methylsulfonyl)-, 1-methylethyl ester, (2S); manumycin A; L-744,832 or DK8G557, tipifarnib (R115777), SCH66336 (Ionafarnib), and BMS-214662,

The term "a Flk-1 kinase inhibitor", as used herein, relates to a compound which targets, decreases or inhibits Flk-1 tyrosine kinase activity. A target of a Flk-1 kinase inhibitor includes, but is not limited to, KDR. An example of a Flk-1 kinase inhibitor includes, but is not limited to, 2-propenamide, 2-cyano-3-[4-hydroxy-3,5-bis(1-methylethyl)phenyl]-N-(3-phenylpropyl)-,(2E).

The term "a Glycogen synthase kinase-3 (GSK3) inhibitor", as used herein, relates to a compound which targets, decreases or inhibits glycogen synthase kinase-3 (GSK3). Glycogen Synthase Kinase-3 (GSK-3; tau protein kinase I), a highly conserved, ubiquitously expressed serine/threonine protein kinase, is involved in the signal transduction cascades of multiple cellular processes. which is a protein kinase that has been shown to be involved in the regulation of a diverse array of cellular functions, including protein synthesis, cell proliferation, cell differentiation, microtubule assembly/disassembly, and apoptosis. An example of a GSK3 inhibitor includes, but is not limited to, indirubin-3'-monooxime.

The term "a histone deacetylase (HDAC) inhibitor", as used herein, relates to a compound which inhibits the histone deacetylase and which possess anti-proliferative activity. This includes but is not limited to compounds disclosed in WO 02/22577, especially N-hydroxy-3-[4-[[(2-hydroxyethyl)[2-(1H-indol-3-yl)ethyl]-amino]methyl]phenyl]-2E-2-propenamide, and N-hydroxy-3-[4-[[[2-(2-methyl-1H-indol-3-yl)-ethyl]-amino]methyl]phenyl]-2E-2-propenamide and pharmaceutically acceptable salts thereof. It further includes Suberoylanilide hydroxamic acid (SAHA); [4-(2-amino-phenylcarbamoyl)-benzyl]-carbamic acid pyridine-3-ylmethyl ester and derivatives thereof; butyric acid, pyroxamide, trichostatin A, Oxamflatin, apicidin, Depsipeptide; and trapoxin.Other examples include depudecin; trapoxin, HC Toxin, which is a cyclic tetrapeptide (cyclo-[prolyl-alynyl-alanyl-2-amino-8-oxo-9,10-epoxydecanoyl]); sodium phenylbutyrate, suberoyl bis-hydroxamic acid, Trichostatin A, BMS-27275, pyroxamide, FR-901228 and valproic acid..

The term "HSP90 inhibitor", as used herein, relates to a compound which targets, decreases or inhibits the intrinsic ATPase activity of HSP90; degrades, targets, decreases or inhibits the HSP90 client proteins via the ubiquitin proteosome pathway. Potential indirect targets of an HSP90 inhibitor include FLT3, BCR-ABL, CHK1, CYP3A5*3 and/or NQ01*2. Compounds targeting, decreasing or inhibiting the intrinsic ATPase activity of HSP90 are especially compounds, proteins or antibodies which inhibit the ATPase activity of HSP90, e.g., 17-allylamino,17-demethoxygeldanamycin (17AAG), a geldanamycin derivative; other geldanamycin-related compounds; radicicol and HDAC inhibitors. Other examples of an HSP90 inhibitor include geldanamycin, 17-demethoxy-17-(2-propenylamino)-(9Cl); and Geldanamycin. Nilotinib is an example of an BCR-ABL tyrosine kinase inhibitor.

The term "a I-kappa B-alpha kinase inhibitor (IKK)", as used herein, relates to a compound which targets, decreases or inhibits NF-kappaB. An example of an IKK inhibitor includes, but is not limited to, 2-propenenitrile, 3-[(4-methylphenyl)sulfonyl]-, (2E).

The term "an insulin receptor tyrosine kinase inhibitor", as used herein, relates to a compound which modulates the activities of phosphatidylinositol 3-kinase, microtubule-associated protein, and S6 kinases. Examples of an insulin receptor tyrosine kinase inhibitor include, but are not limited to, hydroxyl-2-naphthalenylmethylphosphonic acid and and LY294002.

The term "a c-Jun N-terminal kinase (JNK) kinase inhibitor", as used herein, relates to a compound which targets, decreases or inhibits Jun N-terminal kinase. Jun N-terminal kinase (JNK), a serine-directed protein kinase, is involved in the phosphorylation and activation of c-Jun and ATF2 and plays a significant role in metabolism, growth, cell differentiation, and apoptosis. A target for a JNK kinase inhibitor includes, but is not limited to, DNMT. Examples of a JNK kinase inhibitor include, but are not limited to, pyrazoleanthrone and/or epigallocatechin gallate.

The term "a microtubule binding agent", as used herein, refers to a compound which acts by disrupting the microtubular network that is essential for mitotic and interphase cellular function. Examples of a microtubule binding agent include, but are not limited to, vinca alkaloids, e.g. vinblastine, vinblastine sulfate; vincristine, vincristine sulfate; vindesine; vinorelbine; taxanes, e.g. docetaxel; paclitaxel; discodermolides; cochicine and epothilones and derivatives thereof, e.g., epothilone B or a derivative thereof. Paclitaxel is marketed as TAXOL®; docetaxel as TAXOTERE®; vinblastine sulfate as VINBLASTIN R.P®; and vincristine sulfate as FARMISTIN®. Also included are the generic forms of paclitaxel as well as various dosage forms of paclitaxel. Generic forms of paclitaxel include, but are not limited to, betaxolol hydrochloride. Various dosage forms of paclitaxel include, but are not limited to albumin nanoparticle paclitaxel marketed as ABRAXANE®; ONXOL®, CYTOTAX®.. Discodermolide can be obtained, e.g., as disclosed in US5010099. Also included are Epotholine derivatives which are disclosed in US6194181, WO9810121, WO9825929, WO9808849, WO9943653, WO9822461 and WO0031247. Especially preferred are Epotholine A and/or B.

The term "a Mitogen-activated protein (MAP) kinase-inhibitor", as used herein, relates to a compound which targets, decreases or inhibits Mitogen-activated protein. The mitogen-activated protein (MAP) kinases are a group of protein serine/threonine kinases that are activated in response to a variety of extracellular stimuli and mediate signal transduction from the cell surface to the nucleus. They regulate several physiological and pathological cellular phenomena, including inflammation, apoptotic cell death, oncogenic transformation, tumor cell invasion, and metastasis. An example of a MAP kinase inhibitor includes, but is not limited to, benzenesulfonamide, N-[2-[[[3-(4-chlorophenyl)-2-propenyl]methyl]amino]methyl]phenyl]-N-(2-hydroxyethyl)-4-methoxy-.

The term "a MDM2 inhibitor", as used herein, relates to a compound which targets, decreases or inhibits the interaction of MDM2 and the p53 tumor suppressor. An example of a a MDM2 inhibitor includes, but is not limited to, trans-4-iodo, 4'-boranyl-chalcone.

The term "a MEK inhibitor", as used herein, relates to a compound which targets, decreases or inhibits the kinase activity of MAP kinase, MEK. A target of a MEK inhibitor includes, but is not limited to, ERK. An indirect target of a MEK inhibitor includes, but is not limited to, cyclin D1. An example of a MEK inhibitor includes, but is not limited to, such as sorafenib, e.g. Nexavar® (sorafenib tosylate), butanedinitrile, bis[amino[2-aminophenyl)thio]methylene].

The term "a MMP inhibitor", as used herein, relates to a compound which targets, decreases or inhibits a class of protease enzyme that selectively catalyze the hydrolysis of polypeptide bonds including the enzymes MMP-2 and MMP-9 that are involved in promoting the loss of tissue structure around tumours and facilitating tumour growth, angiogenesis, and metastasis. A target of a MMP inhibitor includes, but is not limited to, polypeptide deformylase. Example of a MMP inhibitor include, but are not limited to, Actinonin, which is also known as Butanediamide, N4-hydroxy-N1-[(1S)-1-[[(2S)-2-(hydroxymethyl)-1-pyrrolidinyl]carbonyl]-2-methylpropyl]-2-pentyl-, (2R); epigallocatechin gallate; collagen peptidomimetic and non-peptidomimetic inhibitors; tetracycline derivatives, e.g., hydroxamate peptidomimetic inhibitor batimastat; and its orally-bioavailable analogue marimastat, prinomastat" metastat, Neovastat, Tanomastat, TAA211, MMI270B, BMS-279251, BAY 12-9566, or AAJ996.

The term "a NGFR tyrosine-kinase-inhibitor", as used herein, relates to a compound which targets, decreases or inhibits nerve growth factor dependent p140^{c*-trk*} tyrosine phosphorylation. Targets of a NGFR tyrosine-kinase-inhibitor include, but are not limited to, HER2, FLK1, FAK, TrkA, and/or TrkC. An indirect target inhibits expression of RAF1. An example of a NGFR tyrosine-kinase-inhibitor includes, but is not limited to, Tyrphostin AG 879.

The term "a p38 MAP kinase inhibitor", as used herein, relates to a compound which targets, decreases or inhibits p38-MAPK, which is a MAPK family member. A MAPK family member is a serine/threonine kinase activated by phosphorylation of tyrosine and threonine residues. This kinase is phosphorylated and activated by many cellular stresses and inflammatory stimuli, thought to be involved in the regulation of important cellular responses such as apoptosis and inflammatory reactions. An example of a a p38 MAP kinase inhibitor includes, but is not limited to, Phenol, 4-[4-(4-fluorophenyl)-5-(4-pyridinyl)-1H-imidazol-2-yl]. An example of a a SAPK2/p38 kinase inhibitor includes, but is not limited to, benzamide, 3-(dimethylamino)-N-[3-[(4-hydroxybenzoyl)amino]-4-methylphenyl].

The term "a p56 tyrosine kinase inhibitor", as used herein, relates to a compound which targets, decreases or inhibits p56 tyrosine kinase, which is an enzyme that is a lymphoid-specific src family tyrosine kinase critical for T-cell development and activation. A target of a p56 tyrosine kinase inhibitor includes, but is not limited to, Lck. Lck is associated with the cytoplasmic domains of CD4, CD8 and the beta-chain of the IL-2 receptor, and is thought to be involved in the earliest steps of TCR-mediated T-cell activation. Examples of a p56 tyrosine kinase inhibitor include, but are not limited to, damnacanthal, which is also known as 2-anthracenecarboxaldehyde,9,10-dihydro-3-hydroxy-1methoxy-9,10-dioxo, and/or Tyrphostin 46.

The term "a PDGFR tyrosine kinase inhibitor", as used herein, relates to compounds targeting, decreasing or inhibiting the activity of the C-kit receptor tyrosine kinases (part of the PDGFR family), such as compounds which target, decrease or inhibit the activity of the c-Kit receptor tyrosine kinase family, especially compounds which inhibit the c-Kit receptor, PDGF plays a central role in regulating cell proliferation, chemotaxis, and survival in normal cells as well as in various disease states such as cancer, atherosclerosis, and fibrotic disease. The PDGF family is composed of dimeric isoforms (PDGF-AA, PDGF-BB, PDGF-AB, PDGF-CC, and PDGF-DD), which exert their cellular effects by differentially binding to two receptor tyrosine kinases. PDGFR-α and PDGFR-ß have molecular masses of ~170 and 180 kDa, respectively.. Examples of targets of a PDGFR tyrosine kinase inhibitor includes, but are not limited to PDGFR, FLT3 and/or c-KIT. Example of a PDGFR tyrosine kinase inhibitor include, but are not limited to, Tyrphostin AG 1296; Tyrphostin 9; 1,3-butadiene-1,1,3-tricarbonitrile,2-amino-4-(1H-indol-5-yl); Imatinib and IRESSA.

The term "a phosphatidylinositol 3-kinase inhibitor", as used herein, relates to a compound which targets, decreases or inhibits PI 3-kinase. PI 3-kinase activity has been shown to increase in response to a number of hormonal and growth factor stimuli, including insulin, platelet-derived growth factor, insulin-like growth factor, epidermal growth factor, colony-stimulating factor, and hepatocyte growth factor, and has been implicated in processes related to cellular growth and transformation. An example of a target of a phosphatidylinositol 3-kinase inhibitor includes, but is not limited to, Pi3K. Examples of a phosphatidylinositol 3-kinase inhibitor include, but are not limited to, Wortmannin, which is also known as 3H-Furo[4,3,2-de]indeno[4,5-h]-2-benzopyran-3,6,9-trione, 11-(acetyloxy)-1,6b,7,8,9a,10,11,11b-octahydro-1-(methoxymethyl)-9a,11b-dimethyl-, (1S,6bR,9aS,11R,11bR); 8-phenyl-2-(morpholin-4-yl)-chromen-4-one; and/or quercetin, e.g. quercentin dihydrate.

The term "a phosphatase inhibitor", as used herein, relates to a compound which targets, decreases or inhibits phosphatase. Phosphatases remove the phosphoryl group and restore the protein to its original dephosphorylated state. Hence, the phosphorylation-dephosphorylation cycle can be regarded as a molecular "on-off" switch. Examples of a phosphatase inhibitor include, but are not limited to, cantharidic acid; cantharidin; and L-leucinamide, N-[4-(2-carboxyethenyl)benzoyl]glycyl-L-α-glutamyl-,(E).

The term "a platinum agent", as used herein, relates to a compound which contains Platinum and inhibit DNA synthesis by forming interstrand and intrastrand cross-linking of DNA molecules. Examples of a a platinum agent include, but are not limited to, Carboplatin; Cisplatin; Oxaliplatin; cisplatinum; Satraplatin and platinum agents such as ZD0473 BBR3464. Carboplatin can be administered, e.g., in the form as it is marketed, e.g. CARBOPLAT®; and oxaliplatin as ELOXATIN®.

The term "a protein phosphatase inhibitor", as used herein, relate to a compound which targets, decreases or inhibits protein phosphatase. The term "a PP1 or PP2 inhibitor", as used herein, relates to a compound which targets, decreases or inhibits Ser/Thr protein phosphatases. Type I phosphatases, which include PP1, can be inhibited by two heat-stable proteins known as Inhibitor-1 (I-1) and Inhibitor-2 (I-2). They preferentially dephosphorylate the 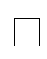 -subunit of phosphorylase kinase. Type II phosphatases are subdivided into spontaneously active (PP2A), CA²⁺-dependent (PP2B), and Mg²⁺-dependent (PP2C) classes of phosphatases. Examples of a PP1 and PP2A inhibitor include, but are not limited to, cantharidic acid and/or cantharidin. The term "tyrosine phosphatase inhibitor", as used here, relates to a compouns which targets, decreases or inhibits tyrosine phosphatase. Protein tyrosine phosphatases (PTPs) are relatively recent additions to the phosphatase family. They remove phosphate groups from phosphorylated tyrosine residues of proteins. PTPs display diverse structural features and play important roles in the regulation of cell proliferation, differentiation, cell adhesion and motility, and cytoskeletal function. Examples of targets of a tyrosine phosphatase inhibitor include, but are not limited to, alkaline phosphatase (ALP), heparanase, PTPase, and/or prostatic acid phosphatase. Examples of a tyrosine phosphatase inhibitor include, but are not limited to, L-P-bromotetramisole oxalate; 2(5H)-furanone,4-hydroxy-5-(hydroxymethyl)-3-(1-oxohexadecyl)-, (5R)-(9Cl); and benzylphosphonic acid.

The term "a PKC inhibitor", as used herein, relates to a compound which targets, decreases or inhibits protein kinase C as well as its isozymes. Protein kinase C (PKC), a ubiquitous, phospholipid-dependent enzyme, is involved in signal transduction associated with cell proliferation, differentiation, and apoptosis. Examples of a target of a PKC inhibitor include, but are not limited to, MAPK and/or NF-kappaB. Examples of a PKC inhibitor include, but are not limited to, 1-H-pyrrolo-2,5-dione,3-[1-[3-(dimethylamino)propyl]-1H-indol-3-yl]-4-(1 H-indol-3-yl); Bisindolylmaleimide IX; Sphingosine, which is known as 4-Octadecene-1,3-diol, 2-amino-, (2S,3R,4E); staurosporine, which is known as 9,13-Epoxy-1H,9H-diindolo[1,2,3-gh:3',2',1'-Im]pyrrolo[3,4-j][1,7]benzodiazonin-1-one, 2,3,10,11,12,13-hexahydro-10-methoxy-9-methyl-11-(methylamino)-, (9S,10R,11R,13R), tyrphostin 51; and Hypericin, which is also known as Phenanthro[1,10,9,8-opqra]perylene-7,14-dione, 1,3,4,6,8,13-hexahydroxy-10,11-dimethyl-, stereoisomer, UCN-01,safingol, BAY 43-9006, bryostatin 1, perifosine;Ilmofosine ; RO 318220, RO 320432; GO 6976 ; Isis 3521; and LY333531/LY379196.

The term "a PKC delta kinase inhibitor", as used herein, relates to a compound which targets, decreases or inhibits the delta isozymes of PKC. The delta isozyme is a conventional PKC isozymes and is Ca²⁺-dependent. An example of a PKC delta kinase inhibitor includes, but is not limited to, Rottlerin, which is also known as 2-Propen-1-one, 1-[6-[(3-acetyl-2,4,6-trihydroxy-5-methylphenyl)methyl]-5,7-dihydroxy-2,2-dimethyl-2H-1-benzopyran-8-yl]-3-phenyl-, (2E).

The term "a polyamine synthesis inhibitor", as used herein, relates to a compound which targets, decreases or inhibits polyamines spermidine. The polyamines spermidine and spermine are of vital importance for cell proliferation, although their precise mechanism of action is unclear. Tumor cells have an altered polyamine homeostasis reflected by increased activity of biosynthetic enzymes and elevated polyamine pools. Examples of a a polyamine synthesis inhibitor include, but are not limited to, DMFO, which is also known as (-)-2-difluoromethylornithin; N1, N12-diethylspermine 4HCl.

The term "a proteosome inhibitor", as used herein, relates to a compound which targets, decreases or inhibits proteasome.. Examples of targets of a proteosome inhibitor include, but are not limited to, O(2)(-)-generating NADPH oxidase, NF-kappaB, and/or farnesyltransferase, geranylgeranyltransferase I. Examples of a proteosome inhibitor include, but are not limited to, aclacinomycin A; gliotoxin; PS-341; MLN 341; bortezomib (Velcade®).

The term "a PTP1 B inhibitor", as used herein, relates to a compound which targets, decreases or inhibits PTP1 B, a protein tyrosine kinase inhibitor. An example of a PTP1 B inhibitor includes, but is not limited to, L-leucinamide, N-[4-(2-carboxyethenyl)benzoyl]glycyl-L-α-glutamyl-,(E).

The term "a protein tyrosine kinase inhibitor", as used herein, relates to a compound which which targets, decreases or inhibits protein tyrosine kinases. Protein tyrosine kinases (PTKs) play a key role in the regulation of cell proliferation, differentiation, metabolism, migration, and survival. They are classified as receptor PTKs and non-receptor PTKs. Receptor PTKs contain a single polypeptide chain with a transmembrane segment. The extracellular end of this segment contains a high affinity ligand-binding domain, while the cytoplasmic end comprises the catalytic core and the regulatory sequences. Examples of targets of a tyrosine kinase inhibitor include, but are not limited to, ERK1, ERK2, Bruton's tyrosine kinase (Btk), JAK2, ERK ½, PDGFR, and/or FLT3. Examples of indirect targets include, but are not limited to, TNFalpha, NO, PGE2, IRAK, iNOS, ICAM-1, and/or E-selectin. Examples of a tyrosine kinase inhibitor include, but are not limited to, Tyrphostin AG 126; Tyrphostin Ag 1288; Tyrphostin Ag 1295; Geldanamycin; and Genistein.

Non-receptor tyrosine kinases include members of the Src, Tec, JAK, Fes, Abl, FAK, Csk, and Syk families. They are located in the cytoplasm as well as in the nucleus. They exhibit distinct kinase regulation, substrate phosphorylation, and function. Deregulation of these kinases has also been linked to several human diseases.

The term "a SRC family tyrosine kinase inhibitor", as used herein, relates to a compound which which targets, decreases or inhibits SRC. Examples of a SRC family tyrosine kinase inhibitor include, but are not limited to, PP1, which is also known as 1 H-Pyrazolo[3,4-d]pyrimidin-4-amine, 1-(1,1-dimethylethyl)-3-(1-naphthalenyl); and PP2, which is also known as 1H-Pyrazolo[3,4-d]pyrimidin-4-amine, 3-(4-chlorophenyl)-1-(1,1-dimethylethyl).

The term "a Syk tyrosine kinase inhibitor", as used herein, relates to a compound which targets, decreases or inhibits Syk. Examples of targets for a Syk tyrosine kinase inhibitor include, but are not limited to, Syk, STAT3, and/or STAT5. An example of a Syk tyrosine kinase inhibitor includes, but is not limited to, Piceatannol, which is also known as 1,2-Benzenediol, 4-[(1 E)-2-(3,5-dihydroxyphenyl)ethenyl].

The term "a Janus (JAK-2 and/or JAK-3) tyrosine kinase inhibitor", as used herein, relates to a compound which targets, decreases or inhibits janus tyrosine kinase. Janus tyrosine kinase inhibitor are shown anti-leukemic agents with anti-thrombotic, anti-allergic and immunosuppressive properties. Targets of a JAK-2 and/or JAK-3 tyrosine kinase inhibitor include, but are not limited to, JAK2, JAK3, STAT3. An indirect target of an JAK-2 and/or JAK-3 tyrosine kinase inhibitor includes, but is not limited to CDK2. Examples of a JAK-2 and/or JAK-3 tyrosine kinase inhibitor include, but are not limited to, Tyrphostin AG 490; and 2-naphthyl vinyl ketone.

Compounds which target, decrease or inhibit the activity of c-Abl family members and their gene fusion products, e. g. include PD180970 ; AG957; or NSC 680410.

The term "a retinoid", as used herein, erfers to compounds that target, decrease or inhibit retinoid dependent receptors. Examples include, but are not limited to isotretinoin, tretinoin, alitretinoin, bexarotene, e.g. including an agent which interact with retinoic acid responsive elements on DNA, such as isotretinoin (13-*cis*-retinoic acid).

The term "a RNA polymerase II elongation inhibitor", as used herein, relates to a compound which targets, decreases or inhibits insulin-stimulated nuclear and cytosolic p70S6 kinase in CHO cells; targets, decreases or inhibits RNA polymerase II transcription, which may be dependent on casein kinase II; and targets, decreases or inhibits germinal vesicle breakdown in bovine oocytes An example of a RNA polymerase II elongation inhibitor includes, but is not limited to, 5,6-dichloro-1-beta-D-ribofuranosylbenzimidazole.

The term "a serine/threonine kinase inhibitor", as used herein, relates to a compound which inhibits serine/threonine kinases. An example of a target of a serine/threonine kinase inhibitor includes, but is not limited to, dsRNA-dependent protein kinase (PKR). Examples of indirect targets of a serine/threonine kinase inhibitor include, but are not limited to, MCP-1, NF-kappaB, elF2alpha, COX2, RANTES, IL8,CYP2A5, IGF-1, CYP2B1, CYP2B2, CYP2H1, ALAS-1, HIF-1, erythropoietin, and/or CYP1A1. An example of a serine/theronin kinase inhibitor includes, but is not limited to, 2-aminopurine, also known as 1H-purin-2-amine.

The term "a sterol biosynthesis inhibitor", as used herein, relates to a compound which inhibits the biosynthesis of sterols such as cholesterol Examples of targets for a sterol biosynthesis inhibitor include, but are not limited to, squalene epoxidase, and CYP2D6. An example of a sterol biosynthesis inhibitor includes, but is not limited to, terbinadine.

The term "a topoisomerase inhibitor", includes a topoisomerase I inhibitor and a topoisomerase II inhibitor. Examples of a topoisomerase I inhibitor include, but are not limited to, topotecan, gimatecan, irinotecan, camptothecian and its analogues, 9-nitrocamptothecin and the macromolecular camptothecin conjugate PNU-166148 (compound A1 in WO 99/17804); 10-hydroxycamptothecin acetate salt; etoposide; idarubicin hydrochloride; irinotecan hydrochloride; teniposide; topotecan , topotecan hydrochloride; doxorubicin; epirubicin, epirubicin hydrochloride; 4'-epidoxorubicin, mitoxantrone, mitoxantrone hydrochloride; daunorubicin, daunorubicin hydrochloride, valrubicin and dasatinib (BMS-354825). Irinotecan can be administered, e.g., in the form as it is marketed, e.g., under the trademark CAMPTOSAR®. Topotecan can be administered, e.g., in the form as it is marketed, e.g., under the trademark HYCAMTIN®.
The term "topoisomerase II inhibitor", as used herein, includes, but is not limited to, the anthracyclines, such as doxorubicin, including liposomal formulation, e.g., CAELYX®, daunorubicin, including liposomal formulation, e.g., DAUNOSOME®, epirubicin, idarubicin and nemorubicin; the anthraquinones mitoxantrone and losoxantrone; and the podophillotoxines etoposide and teniposide. Etoposide is marketed as ETOPOPHOS®; teniposide as VM 26-BRISTOL®; doxorubicin as ADRIBLASTIN® or ADRIAMYCIN®; epirubicin as FARMORUBICIN® idarubicin as ZAVEDOS®; and mitoxantrone as NOVANTRON®.

The term "VEGFR tyrosine kinase inhibitor", as used herein, relates to a compound which targets, decreases and/or inhibits the known angiogenic growth factors and cytokines implicated in the modulation of normal and pathological angiogenesis. The VEGF family (VEGF-A, VEGF-B, VEGF-C, VEGF-D) and their corresponding receptor tyrosine kinases [VEGFR-1 (Flt-1), VEGFR-2 (Flk-1, KDR), and VEGFR-3 (Flt-4)] play a paramount and indispensable role in regulating the multiple facets of the angiogenic and lymphangiogenic processes. An example of a VEGFR tyrosine kinase inhibitor includes, but is not limited to, 3-(4-dimethylaminobenzylidenyl)-2-indolinone.

Compounds which target, decrease or inhibit the activity of VEGFR are especially compounds, proteins or antibodies which inhibit the VEGF receptor tyrosine kinase, inhibit a VEGF receptor or bind to VEGF, and are in particular those compounds, proteins or monoclonal antibodies generically and specifically disclosed in WO9835958, e. g.1- (4-chloroanilino)-4- (4-pyridylmethyl) phthalazine or a pharmaceutical acceptable salt thereof, e. g. the succinate, or in WO0009495, WO0027820, WO0059509, WO9811223, WO0027819 and EP0769947; e.g. those as described by M. Prewett et al in Cancer Research 59 (1999) 5209-5218, by F. Yuan et al in Proc. Natl. Acad. Sci. USA, vol. 93, pp. 14765-14770, Dec. 1996, by Z. Zhu et al in Cancer Res. 58,1998,3209-3214, and by J. Mordenti et al in Toxicologic Pathology, Vol. 27, no. 1, pp 14-21,1999; in WO0037502 and WO9410202; Angiostatin, described by M. S. O'Reilly et al, Cell 79,1994,315-328; Endostatin described by M. S. O'Reilly et al, Cell 88,1997,277-285;anthranilic acid amides; ZD4190; ZD6474 (vandetanib); SU5416; SU6668, AZD2171 (Recentin®); or anti-VEGF antibodies, such as anti-VEGF-alpha antibody tanibizumab (Lucentis®), or anti-VEGF receptor antibodies, e. g. RhuMab (bevacizumab, Avastin®). By antibody is meant intact monoclonal antibodies, polyclonal antibodies, multispecific antibodies formed from at least 2 intact antibodies, and antibodies fragments so long as they exhibit the desired biological activity. an example of an VEGF-R2 inhibitor e.g. includes axitinib,

The term "gonadorelin agonist" e.g. includes compounds such as abarelix, goserelin, goserelin acetate (ZOLADEX®).

The term "a compound which induce cell differentiation processes" includes compounds such as retinoic acid, alpha-, gamma- or 8- tocopherol or alpha-, gamma- or 8-tocotrienol.

The term "a bisphosphonate" includes but is not limited to etridonic, clodronic, tiludronic, pamidronic, alendronic, ibandronic, risedronic and zoledronic acid.

The term "a heparanase inhibitor" is a compound which prevents heparan sulphate degradation, including but not limited to PI-88.

The term "a biological response modifier" includes but is not limited to ymphokines or interferons, e. g. interferon alpha,

The term "a telomerase inhibitor" includes inhibitors of telomerase activity, including but not limited to telomestatin. Telomerase is attracting great interest as a target for anticancer research because telomerase activity is present in most malignant cells.

The term "mediators, such as inhibitors, of catechol-O-methyltransferase" includes but is not limited to entacapone.

The term "an immunosuppressive monoclonal antibody, e.g. monoclonal, to leukocyte receptors or their ligands" includes but is not limited to antibodies to
- CD20, such as rituximab (Rituxan®, ibritumomab tiuxetan conjugated to ¹¹¹In or ⁹⁰Y (Zevalin®), ¹³¹I tositumumab ()Bexxar®), ofatumumab, ocrelizumab, hA20 (Immunomedics),
- CD22, such as epratuzumab, inotizumab ozogamicin (CMC544), CAT-3888,
- CD33, such as gemtuzumab (Mylotarg®,
- CD52, e.g. alemtuzumab (Campath-I®),
- CD11 a, e.g. efalizumab (Raptiva®), and
- CD3, e.g. visillzumab.

The term "somatostatin or a somatostatin analogue" includes but is not limited to octreotide (Sandostatin® or Sandostatin LAR®).

The term "Growth Hormone-Receptor Antagonists", includes but is not limited to pegvisomant, filgrastim or pegfilgrastim, or interferon alpha:
The term "monoclonal antibodies useful for leukemia (AML) treatment" includes but is not limited to alemtuzumab (Campath ®), rituximab /Rituxan®), gemtuzumab, (ozogamicin, Mylotarg®),.epratuzumab.

The term "antibodies against carcinoembryonic antigen (CEA)" includes but is not limited to lapetuzumab, e.g. lapetuzumab-yttrium90, KSB-303, MFECP1, MFE-23.

The term "a phosphodiesterase inhibitor" includes but is not limited to anagrelide (Agrylin®, Xagrid®).

The term "a cancer vaccine" includes vaccines which are useful for cancer treatment/prevention and includes but is not limited to MDX-1379.

The term "inhibitors of Kinesin Spindle Protein (KSP) includes but is not limited to ispinesib.

The term "inhibitors of multiple receptor tyrosine kinases associated with tumour growth and angiogenesis" includes but is not limited to sunitinib (SU11248).

The term "synthetic nonsteroidal estrogens" includes but is not limited to diethylstilbestrol (DES, Stilboestrol®)).

The term "cytoxic antineoplastics" includes but is not limited to altretamine, amsacrine, asparaginase (Elspar®), pegaspargase (PEG-L-asparaginase, Oncaspar®)), denileukin diftitox (Ontak®)) and masoprocol,

The term "a recombinant binding molecule having at least a portion of the extracellular domain of CTLA4 or a mutant thereof, or an anti-CLA4 agent" e.g. including an at least extracellular portion of CTLA4 or a mutant thereof joined to a non-CTLA4 protein sequence, such as CTLA4Ig, (e.g. designated ATCC 68629) or a mutant thereof includes but is not limited to LEA29Y (belatacept); an anti-CTLA4 agent includes but is not limited to ipilimumab, ticilimumab.

In each case where citations of patent applications or scientific publications are given, in particular with regard to the respective compound claims and the final products of the working examples therein, the subject matter of the final products, the pharmaceutical preparations and the claims is hereby incorporated into the present application by reference to these publications. Comprised are likewise the corresponding salts, stereoisomers, as well as corresponding crystal modifications, e.g., solvates and polymorphs, e.g. such as disclosed therein. The compounds used as active ingredients in the combinations disclosed herein can be prepared and administered as described in the cited documents, respectively.

The structure of the active agents identified by code numbers, generic or trade names may be taken from the actual edition of the standard compendium "The Merck Index" or from databases, e.g., Patents International, e.g., IMS World Publications, the INTERNET, or the publications mentioned above and below. The corresponding content thereof is hereby incorporated by reference.

It will be understood that references to the components (a) and (b) are meant to also include the pharmaceutically acceptable salts of any of the active substances. If active substances comprised by components (a) and/or (b) have, for example, at least one basic center, they can form acid addition salts. Corresponding acid addition salts can also be formed having, if desired, an additionally present basic center. Active substances having an acid group, e.g., COOH, can form salts with bases. The active substances comprised in components (a) and/or (b) or a pharmaceutically acceptable salts thereof may also be used in form of a solvate, such as a hydrate or other solvates originating from solvents used for crystallization. 40-O-(2-hydroxyethyl)-rapamycin, is the most preferred combination partner (a).

### III. The Combinations

The present invention relates to a combination of:
(a) a mTOR inhibitor compound; and
(b) an pharmaceutically active agent.

In preferred embodiment, the present invention provides a combination comprising:
(a) a mTOR inhibitor compound; and
(b) one or more pharmaceutically active agents selected from the group consisting of a steroid, an adenosine-kinase-inhibitor; an adjuvant; an adrenal cortex antagonist; AKT pathway inhibitor; an alkylating agent; an angiogenesis inhibitor; an anti-androgen; an anti-estrogen; an anti-hypercalcemia agent; an antimetabolite; an apoptosis inducer; an aurora kinase inhibitor; a Bruton's Tyrosine Kinase (BTK) inhibitor; a calcineurin inhibitor; a CaM kinase II inhibitor; a CD45 tyrosine phosphatase inhibitor; a CDC25 phosphatase inhibitor; a CHK kinase inhibitor; a controlling agent for regulating genistein, olomucine and/or tyrphostins; a cyclooxygenase inhibitor; a cRAF kinase inhibitor; a cyclin dependent kinase inhibitor; a cysteine protease inhibitor; a DNA intercalator; a DNA strand breaker; an E3 Ligase inhibitor; an endocrine hormone; compounds targeting, decreasing or inhibiting the activity of the epidermal growth factor family; an EGFR, PDGFR tyrosine kinase inhibitor; a farnesyltransferase inhibitor; a Flk-1 kinase inhibitor; a Glycogen synthase kinase-3 (GSK3) inhibitor; a histone deacetylase (HDAC) inhibitor; a HSP90 inhibitor; a I-kappa B-alpha kinase inhibitor (IKK); an insulin receptor tyrosine kinase inhibitor; a c-Jun N-terminal kinase (JNK) kinase inhibitor; a microtubule binding agent; a Mitogen-activated protein (MAP) kinase-inhibitor; a MDM2 inhibitor; a MEK inhibitor; a matrix metalloproteinase inhibitor (MMP) inhibitor; a NGFR tyrosine-kinase-inhibitor; a p38 MAP kinase inhibitor, including a SAPK2/p38 kinase inhibitor; a p56 tyrosine kinase inhibitor; a PDGFR tyrosine kinase inhibitor; a phosphatidylinositol 3-kinase inhibitor; a phosphatase inhibitor; a platinum agent; a protein phosphatase inhibitor, including a PP1 and PP2 inhibitor and a tyrosine phosphatase inhibitor; a PKC inhibitor and a PKC delta kinase inhibitor; a polyamine synthesis inhibitor; a proteosome inhibitor; a PTP1 B inhibitor; a protein tyrosine kinase inhibitor including a SRC family tyrosine kinase inhibitor; a Syk tyrosine kinase inhibitor; and a JAK-2 and/or JAK-3 tyrosine kinase inhibitor; a retinoid; a RNA polymerase II elongation inhibitor; a serine/threonine kinase inhibitor; a sterol biosynthesis inhibitor; a topoisomerase inhibitor; and VEGFR tyrosine kinase inhibitor, a gonadorelin agonist, a compound which induce cell differentiation processes, a bisphosphonate, a heparanase inhibitor, a biological response modifier, a telomerase inhibitor, mediators, such as inhibitors of catechol-O-methyltransferase, an immunosuppressive monoclonal antibody, e.g. monoclonal, to leukocyte receptors, somatostatin or a somatostatin analogue, Growth Hormone-Receptor Antagonists,:monoclonal antibodies useful for leukemia (AML) treatment, antibodies against carcinoembryonic antigen (CEA), a phosphodiesterase inhibitor, a cancer vaccine, inhibitors of Kinesin Spindle Protein (KSP), inhibitors of multiple receptor tyrosine kinases associated with tumour growth and angiogenesis, synthetic nonsteroidal estrogens, cytoxic antineoplastics, and a recombinant binding molecule having at least a portion of the extracellular domain of CTLA4 or a mutant thereof.

In another preferred embodiment, the present invention provides a combination comprising:
(a) a mTOR inhibitor compound; and
(b) one or more pharmaceutically active agents selected from the group consisting of Prednisone. 5-lodotubercidin; Leucovorin; Levamisole; Mitotane; Deguelin; Trciribine; KP372-1 (QLT394), Chlorambucil; chlormethine, cyclophosphamide, ifosfamide, melphalan, estramustine; Carmustine; fotemustine, lomustine, Streptozocin, Streptozotocin, Dacarbazine; Procarbazine; Procarbazine hydrochloride, Mechlorethamine, Mechlorethamine hydrochloride, Thiotepa; Temozolomide; Mitomycin; Altretamine; Busulfan; nitrosourea; estramustine, uramustine, Gliadel; nitrogen mustard, Fumagillin; Shikonin; Tranilast; ursolic acid; suramin; bengamide, TNP-470, thalidomide, Nilutamide; bicalutamide; Toremifene; Letrozole; Testolactone; Anastrozole; Bicalutamide; Flutamide; tamoxifen, tamoxifen citrate; Exemestane; fulvestrant; raloxifene; raloxifene hydrochloride; atamestane, formestane, aminoglutethimide, roglethimide, pyridoglutethimide, trilostane, ketokonazole, vorozole, fadrozole, gallium (III) nitrate hydrate; pamidronate disodium; 6-mercaptopurine; Cytarabine; Fludarabine; Flexuridine; Fluorouracil; 5-Fluorouracil, Floxuridine, Capecitabine; Raltitrexed; Methotrexate; Cladribine; Gemcitabine; Gemcitabine hydrochloride; thioguanine 6-thioguanine, Hydroxyurea; 5-azacytidine, decitabine; edatrexate; Permetrexed, Pemetrexed, clofarabine; nelarabine, tiazofurin, pentostatin, deoxycoformycin; fluoromethylene deoxycitidine, 5-aza-2'-deoxycytidine, troxacitabine, ethanol, 2-[[3-(2,3-dichlorophenoxy)propyl]amino]; gambogic acid; Embelin; Arsenic Trioxide; Binucleine 2; terreic acid; Cypermethrin; Deltamethrin; Fenvalerate; Tyrphostin 8; 5-Isoquinolinesulfonic acid, 4-[(2S)-2-[(5-isoquinolinylsulfonyl)methylamino]-3-oxo-3-(4-phenyl-1-piperazinyl)propyl]phenyl ester; benzenesulfonamide, N-[2-[[[3-(4-chlorophenyl)-2-propenyl]methyl]amino]methyl]phenyl]-N-(2-hydroxyethyl)-4-methoxy; Phosphonic acid, [[2-(4-bromophenoxy)-5-nitrophenyl]hydroxymethyl]; 1,4-naphthalenedione, 2,3-bis[(2-hydroyethyl)thio]; Debromohymenialdisine; Daidzein; Iso-Olomoucine, Tyrphostin 1, 1H-indole-3-acetamide, 1-(4-chlorobenzoyl)-5-methoxy-2-methyl-N-(2-phenylethyl); 5-alkyl substituted 2-arylaminophenylacetic acid and derivatives, celecoxib, rofecoxib, etoricoxib, valdecoxib; a 5-alkyl-2-arylaminophenylacetic acid, 5-methyl-2-(2'-chloro-6'-fluoroanilino)-phenyl acetic acid, lumiracoxib; 3-(3,5-dibromo-4-hydroxybenzylidene)-5-iodo-1,3-dihydroindol-2-one; and benzamide, 3-(dimethylamino)-N-[3-[(4-hydroxybenzoyl)amino]-4-methylphenyl]; N9-Isopropyl-Olomoucine; Olomoucine; Purvalanol B, Roascovitine; Indirubin, Kenpaullone, purvalanol A, Indirubin-3'-monooxime, 4-morpholinecarboxamide, N-[(1S)-3-fluoro-2-oxo-1-(2-phenylethyl)propyl]amino]-2-oxo-1-(phenylmethyl)ethyl], Plicamycin; Dactinomycin; Bleomycin; N-((3,3,3-trifluoro-2-trifluoromethyl)propionyl)sulfanilamide; Leuprolide; megestrol, megestrol acetate; lapatinib, lapatinib ditosylate; panituzumab, trastuzumab, cetuximab, Iressa, OSI-774, CI-1033, EKB-569, GW-2016, 7H-pyrrolo-[2,3-d]pyrimidine, vatanalib,erlotinib, gefitinib, zalutumumab, Tyrphostin 23, Tyrphostin 25, Tyrphostin 47, Tyrphostin 51 and Tyrphostin AG 825, 2-propenamide, 2-cyano-3-(3,4-dihydroxyphenyl)-N-phenyl-,(2E); Tyrphostin Ag 1478; Lavendustin A; 3-pyridineacetonitrile, α-[(3,5-dichlorophenyl)methylene]-, (αZ), Tyrphostin 46, hydroxyfarnesylphosphonic acid; butanoic acid, 2-[[(2S)-2-[[(2S,3S)-2-[[(2R)-2-amino-3-mercaptopropyl]amino]-3-methylpentyl]oxy]-1-oxo-3-phenylpropyl]amino]-4-(methylsulfonyl) 1-methylethyl ester, (2S); manumycin A; L-744,832, DK8G557, tipifarnib, lonafarnib, BMS-214662, 2-propenamide, 2-cyano-3-[4-hydroxy-3,5-bis(1-methylethyl)phenyl]-N-(3-phenylpropyl)-,(2E); indirubin-3'-monooxime; N-hydroxy-3-[4-[[(2-hydroxyethyl)[2-(1H-indol-3-yl)ethyl]-amino]methyl]phenyl]-2E-2-propenamide, N-hydroxy-3-[4-[[[2-(2-methyl-1H-indol-3-yl)-ethyl]-amino]methyl]phenyl]-2E-2-propenamide, Suberoylanilide hydroxamic acid; [4-(2-amino-phenylcarbamoyl)-benzyl]-carbamic acid pyridine-3-ylmethyl ester and derivatives thereof; butyric acid, pyroxamide, Oxamflatin, apicidin, Depsipeptide; trapoxin, depudecin; trapoxin, a cyclic tetrapeptide (cyclo-[prolyl-alynyl-alanyl-2-amino-8-oxo-9,10-epoxydecanoyl]); HC Toxin, sodium phenylbutyrate, suberoyl bis-hydroxamic acid, Trichostatin A, BMS-27275, pyroxamide, FR-901228, valproic acid, geldanamycin, geldanamycin derivatives; 17-allylamino,17-demethoxygeldanamycin (17AAG), 17-demethoxy-17-(2-propenylamino)-geldanamycin, radicicol., nilotinib, 2-propenenitrile, 3-[(4-methylphenyl)sulfonyl]-(2E), hydroxyl-2-naphthalenylmethylphosphonic acid; LY294002, pyrazoleanthrone; epigallocatechin gallate; vinblastine, vinblastine sulfate; vincristine, vincristine sulfate; vindesine; vinorelbine; docetaxel; paclitaxel; discodermolides; cochicine and epothilones and derivatives thereof, epothilone B or a derivative thereof, ; benzenesulfonamide, N-[2-[[[3-(4-chlorophenyl)-2-propenyl]methyl]amino]methyl]phenyl]-N-(2-hydroxyethyl)-4-methoxy; trans-4-iodo, 4'-boranyl-chalcone; sorafenib, sorafenib tosylate, butanedinitrile, bis[amino[2-aminophenyl)thio]methylene], Actinonin, epigallocatechin gallate; batimastat; marimastat, prinomastat,, metastat, Neovastat, Tanomastat, TAA211, MMI270B, BMS-279251, BAY 12-9566, AAJ996, MMI270B; Tyrphostin AG 879; Phenol, 4-[4-(4-fluorophenyl)-5-(4-pyridinyl)-1H-imidazol-2-yl]; benzamide, 3-(dimethylamino)-N-[3-[(4-hydroxybenzoyl)amino]-4-methylphenyl]; 2-anthracenecarboxaldehyde,9,10-dihydro-3-hydroxy-1methoxy-9,10-dioxo, Tyrphostin 46; Tyrphostin AG 1296; Tyrphostin 9; 1,3-butadiene-1,1,3-tricarbonitrile,2-amino-4-(1H-indol-5-yl); Imatinib, IRESSA®, Wortmannin, quercetin, quercentin dehydrate, cantharidic acid; cantharidin; and L-leucinamide, N-[4-(2-carboxyethenyl)benzoyl]glycyl-L-α-glutamyl-,(E), Carboplatin; Cisplatin; Oxaliplatin; cisplatinum; Satraplatin ZD0473, BBR3464, L-P-bromotetramisole oxalate; 2(5H)-furanone,4-hydroxy-5-(hydroxymethyl)-3-(1-oxohexadecyl)-, (5R), benzylphosphonic acid; 1-H-pyrrolo-2,5-dione,3-[1-[3-(dimethylamino)propyl]-1H-indol-3-yl]-4-(1 H-indol-3-yl); Bisindolylmaleimide IX; Sphingosine, staurosporine, tyrphostin 51; Hypericin, UCN-01, safingol, BAY 43-9006, bryostatin 1, perifosine; Ilmofosine ; RO 318220, RO 320432; GO 6976 ; Isis 3521; LY333531/LY379196, Rottlerin; DMFO; aclacinomycin A; gliotoxin; PS-341; MLN 341; bortezomib, Velcade®; L-leucinamide, N-[4-(2-carboxyethenyl)benzoyl]glycyl-L-α-glutamyl-,(E); Tyrphostin AG 126; Tyrphostin Ag 1288; Tyrphostin Ag 1295; Geldanamycin; Genistein, PP1; PP2; piceatannol, Tyrphostin AG 490; 2-naphthyl vinyl ketone; PD180970 ; AG957; NSC 680410, isotretinoin, tretinoin, alitretinoin, bexarotene, isotretinoin (13-*cis-*retinoic acid), 5,6-dichloro-1-beta-D-ribofuranosylbenzimidazole; 2-aminopurine; terbinadine; gimatecan, irinotecan, camptothecian, 9-nitrocamptothecin PNU-166148, 10-hydroxycamptothecin acetate salt; etoposide; idarubicin, idarubicin hydrochloride; irinotecan, irinotecan hydrochloride; teniposide; topotecan , topotecan hydrochloride; doxorubicin; epirubicin, epirubicin hydrochloride; 4'-epidoxorubicin, mitoxantrone, mitoxantrone hydrochloride; daunorubicin, daunorubicin hydrochloride, valrubicin, dasatinib, BMS-354825,. doxorubicin, CAELYX®, DAUNOSOME®, nemorubicin; mitoxantrone; losoxantrone; etoposide; teniposide; 3-(4-dimethylaminobenzylidenyl)-2-indolinone, 1- (4- chloroanilino)-4-(4-pyridylmethyl) phthalazine, Angiostatin, ZD4190; ZD6474, vandetanib; SU5416; SU6668, AZD2171, Recentin®; tanibizumab, bevacizumab, Avastin®), abarelix, goserelin, goserelin acetate, retinoic acid, alpha-, gamma- or 8- tocopherol or alpha-, gamma- or 8-tocotrienol. etridonic, clodronic, tiludronic, pamidronic, alendronic, ibandronic, risedronic and zoledronic acid, PI-88, interferon alpha, telomestatin, entacapone, rituximab, ibritumomab tiuxetan conjugated to ¹¹¹In or ⁹⁰Y, ¹³¹I tositumumab, ofatumumab, ocrelizumab, hA20 from Immunomedics, epratuzumab, inotizumab ozogamicin, CAT-3888, gemtuzumab, alemtuzumab, efalizumab, visillzumab, octreotide, Sandostatin® , Sandostatin LAR®), pegvisomant, filgrastim or pegfilgrastim, alemtuzumab, epratuzumab, lapetuzumab, lapetuzumab-yttrium90, KSB-303, MFECP1, MFE-23, anagrelide, MDX-1379, ispinesib, sunitinib, diethylstilbestrol, altretamine, amsacrine, asparaginase, pegaspargase, denileukin diftitox, masoprocoll, LEA29Y, belatacept; ipilimumab and ticilimumab.

In preferred embodiment, the present invention provides a combination comprising:
(a) a mTOR inhibitor compound of formula I; and
(b) one or more pharmaceutically active agents selected from the group consisting of a steroid, an alkylating agent; an antimetabolite; a microtubule binding agent; a phosphatidylinositol 3-kinase inhibitor; a platinum agent; a polyamine synthesis inhibitor; a SRC family tyrosine kinase inhibitor; and a topoisomerase inhibitor.

In another preferred embodiment, the present invention provides a combination comprising:
(a) a mTOR inhibitor compound of formula I; and
(b) one or more pharmaceutically active agents selected from the group consisting of prednisone, Thiotepa; N1, N12-diethylspermine 4HCl , 8-phenyl-2-(morpholin-4-yl)-chromen-4-one, 1H-pyrazolo-[3,4-d] pyrimidin-4-amine, 3-(4-chlorophenyl)-1-(1,1-dimethylethyl)-(9Cl), Cytarabine; Cladribine; Vindesine sulfate; Vinorelbine; Carboplatin; idarubicin hydrochloride; and mitoxantrone hydrochloride.

In preferred embodiment, the present invention provides a combination comprising:
(a) 40-O-(2-hydroxyethyl)-rapamycin; and
(b) one or more pharmaceutically active agents selected from the group consisting of a steroid, an alkylating agent; an antimetabolite; a microtubule binding agent; a phosphatidylinositol 3-kinase inhibitor; a platinum agent; a polyamine synthesis inhibitor; a SRC family tyrosine kinase inhibitor; and a topoisomerase inhibitor.

In another preferred embodiment, the present invention provides a combination comprising:
(a) 40-O-(2-hydroxyethyl)rapamycin; and
(b) one or more pharmaceutically active agents selected from the group consisting of prednisone, Thiotepa; N1, N12-diethylspermine 4HCl , 8-phenyl-2-(morpholin-4-yl)-chromen-4-one, 1H-pyrazolo-[3,4-d] pyrimidin-4-amine, 3-(4-chlorophenyl)-1-(1,1-dimethylethyl)-(9Cl), Cytarabine; Cladribine; Vindesine sulfate; Vinorelbine; Carboplatin; idarubicin hydrochloride; and mitoxantrone hydrochloride.

Any of the combination of components (a) and (b), the method of treating a warm-blooded animal comprising administering these two components, a pharmaceutical composition comprising these two components for simultaneous, separate or sequential use, the use of the combination for the delay of progression or the treatment of a proliferative disease or for the manufacture of a pharmaceutical preparation for these purposes or a commercial product comprising such a combination of components (a) and (b), all as mentioned or defined above, will be referred to subsequently also as COMBINATION OF THE INVENTION (so that this term refers to each of these embodiments which thus can replace this term where appropriate).

In another aspect the present invention provides the use of a combination provided by the present invention

### IV. Administration

Simultaneous administration may, e.g., take place in the form of one fixed combination with two or more active ingredients, or by simultaneously administering two or more active ingredients that are formulated independently. Sequential use (administration) preferably means administration of one (or more) components of a combination at one time point, other components at a different time point, that is, in a chronically staggered manner, preferably such that the combination shows more efficiency than the single compounds administered independently (especially showing synergism). Separate use (administration) preferably means administration of the components of the combination independently of each other at different time points, preferably meaning that the components (a) and (b) are administered such that no overlap of measurable blood levels of both compounds are present in an overlapping manner (at the same time).

Also combinations of two or more of sequential, separate and simultaneous administration are possible, preferably such that the combination component-drugs show a joint therapeutic effect that exceeds the effect found when the combination component-drugs are used independently at time intervals so large that no mutual effect on their therapeutic efficiency can be found, a synergistic effect being especially preferred.

The term "delay of progression" as used herein means administration of the combination to patients being in a pre-stage or in an early phase, of the first manifestation or a relapse of the disease to be treated, in which patients, e.g., a pre-form of the corresponding disease is diagnosed or which patients are in a condition, e.g., during a medical treatment or a condition resulting from an accident, under which it is likely that a corresponding disease will develop.

"Jointly therapeutically active" or "joint therapeutic effect" means that the compounds may be given separately (in a chronically staggered manner, especially a sequence-specific manner) in such time intervals that they preferably, in the warm-blooded animal, especially human, to be treated, still show a (preferably synergistic) interaction (joint therapeutic effect). Whether this is the case, can inter alia be determined by following the blood levels, showing that both compounds are present in the blood of the human to be treated at least during certain time intervals.

"Pharmaceutically effective" preferably relates to an amount that is therapeutically or in a broader sense also prophylactically effective against the progression of a proliferative disease.

### V. Commercial Package

The term "a commercial package" or "a product" or "a pharmaceutical package", as used herein defines especially a "kit of parts" in the sense that the components (a) and (b) as defined above can be dosed independently or by use of different fixed combinations with distinguished amounts of the components (a) and (b), i.e., simultaneously or at different time points. Moreover, these terms comprise a commercial package comprising (especially combining) as active ingredients components (a) and (b), together with instructions for simultaneous, sequential (chronically staggered, in time-specific sequence, preferentially) or (less preferably) separate use thereof in the delay of progression or treatment of a proliferative disease. The parts of the kit of parts can then, e.g., be administered simultaneously or chronologically staggered, that is at different time points and with equal or different time intervals for any part of the kit of parts. Very preferably, the time intervals are chosen such that the effect on the treated disease in the combined use of the parts is larger than the effect which would be obtained by use of only any one of the combination partners (a) and (b) (as can be determined according to standard methods. The ratio of the total amounts of the combination partner (a) to the combination partner (b) to be administered in the combined preparation can be varied, e.g., in order to cope with the needs of a patient sub-population to be treated or the needs of the single patient which different needs can be due to the particular disease, age, sex, body weight, etc. of the patients. Preferably, there is at least one beneficial effect, e.g., a mutual enhancing of the effect of the combination partners (a) and (b), in particular a more than additive effect, which hence could be achieved with lower doses of each of the combined drugs, respectively, than tolerable in the case of treatment with the individual drugs only without combination, producing additional advantageous effects, e.g., less side effects or a combined therapeutic effect in a non-effective dosage of one or both of the combination partners (components) (a) and (b), and very preferably a strong synergism of the combination partners (a) and (b).

Both in the case of the use of the combination of components (a) and (b) and of the commercial package, any combination of simultaneous, sequential and separate use is also possible, meaning that the components (a) and (b) may be administered at one time point simultaneously, followed by administration of only one component with lower host toxicity either chronically, e.g., more than 3-4 weeks of daily dosing, at a later time point and subsequently the other component or the combination of both components at a still later time point (in subsequent drug combination treatment courses for an optimal anti-tumor effect) or the like.

The COMBINATION OF THE INVENTION can also be applied in combination with other treatments, e.g. such as usual in preventing or treating proliferative diseases, such as surgical intervention, hyperthermia and/or irradiation therapy.

### VI. Pharmaceutical Compositions & Preparations

The pharmaceutical compositions according to the present invention can be prepared by conventional means and are those suitable for enteral, such as oral or rectal, and parenteral administration to mammals including man, comprising a therapeutically effective amount of a VEGF inhibitor and at least one pharmaceutically active agent alone or in combination with one or more pharmaceutically acceptable carriers, especially those suitable for enteral or parenteral application.

The pharmaceutical compositions comprise from about 0.00002 to about 100%, especially, e.g., in the case of infusion dilutions that are ready for use) of 0.0001 to 0.02%, or, e.g., in case of injection or infusion concentrates or especially parenteral formulations, from about 0.1% to about 95%, preferably from about 1% to about 90%, more preferably from about 20% to about 60% active ingredient (weight by weight, in each case). Pharmaceutical compositions according to the invention may be, e.g., in unit dose form, such as in the form of ampoules, vials, dragées, tablets, infusion bags or capsules.

The effective dosage of each of the combination partners employed in a formulation of the present invention may vary depending on the particular compound or pharmaceutical compositions employed, the mode of administration, the condition being treated and the severity of the condition being treated. A physician, clinician or veterinarian of ordinary skill can readily determine the effective amount of each of the active ingredients necessary to prevent, treat or inhibit the progress of the condition.

However, in general, for satisfactory results in larger mammals, for example humans, an indicated daily dosage includes a range
- from about 0.0001 g to about 1.5 g, such as 0.001 g to 1.5 g;
- from about 0.001 mg/kg body weight to about 20 mg/kg body weight, such as 0.01 mg/kg body weight to 20 mg/kg body weight,
for example administered in divided doses up to four times a day.

In a combination of the present invention, rapamycin or a rapaymcin derivative may be administered as appropriate, e.g. in dosages which are known for compounds of the present invention, by any administration route, e.g. enterally, e.g. orally, or parenterally. E.g. everolimus may be administered, e.g. orally, in dosages from 0.1 mg up to 15 mg, such as 0.1 mg to 10 mg. e.g. 0.1 mg, 0.25 mg, 0.5 mg, 0.75 mg, 1 mg, 2.5 mg, 5 mg, or 10 mg, more preferably from 0.5 mg to 10 mg, e.g. in the form of (dispersible) tablets; e.g. comprising everolimus in the form of a solid dispersion; e.g. a weekly dosage may include up to 70 mg, e.g. 10 to 70 mg, such as 30 to 50 mg, e.g. depending on the disease being treated.

Pharmaceutical preparations for the combination therapy for enteral or parenteral administration are, e.g., those in unit dosage forms, such as sugar-coated tablets, capsules or suppositories, and furthermore ampoules. If not indicated otherwise, these formulations are prepared by conventional means, e.g., by means of conventional mixing, granulating, sugar-coating, dissolving or lyophilizing processes. It will be appreciated that the unit content of a combination partner contained in an individual dose of each dosage form need not in itself constitute an effective amount since the necessary effective amount can be reached by administration of a plurality of dosage units. One of skill in the art has the ability to determine appropriate pharmaceutically effective amounts of the combination components.

Preferably, the compounds or the pharmaceutically acceptable salts thereof, are administered as an oral pharmaceutical formulation in the form of a tablet, capsule or syrup; or as parenteral injections if appropriate.

In preparing compositions for oral administration, any pharmaceutically acceptable media may be employed such as water, glycols, oils, alcohols, flavoring agents, preservatives, coloring agents. Pharmaceutically acceptable carriers include starches, sugars, microcrystalline celluloses, diluents, granulating agents, lubricants, binders, disintegrating agents.

Solutions of the active ingredient, and also suspensions, and especially isotonic aqueous solutions or suspensions, are useful for parenteral administration of the active ingredient, it being possible, e.g., in the case of lyophilized compositions that comprise the active ingredient alone or together with a pharmaceutically acceptable carrier, e.g., mannitol, for such solutions or suspensions to be produced prior to use. The pharmaceutical compositions may be sterilized and/or may comprise excipients, e.g., preservatives, stabilizers, wetting and/or emulsifying agents, solubilizers, salts for regulating the osmotic pressure and/or buffers, and are prepared in a manner known per se, e.g., by means of conventional dissolving or lyophilizing processes. The solutions or suspensions may comprise viscosity-increasing substances, such as sodium carboxymethylcellulose, carboxymethylcellulose, dextran, polyvinylpyrrolidone or gelatin. Suspensions in oil comprise as the oil component the vegetable, synthetic or semi-synthetic oils customary for injection purposes.

The isotonic agent may be selected from any of those known in the art, e.g. mannitol, dextrose, glucose and sodium chloride. The infusion formulation may be diluted with the aqueous medium. The amount of aqueous medium employed as a diluent is chosen according to the desired concentration of active ingredient in the infusion solution. Infusion solutions may contain other excipients commonly employed in formulations to be administered intravenously such as antioxidants.

The present invention further relates to "a combined preparation", which, as used herein, defines especially a "kit of parts" in the sense that the combination partners (a) and (b) as defined above can be dosed independently or by use of different fixed combinations with distinguished amounts of the combination partners (a) and (b), i.e., simultaneously or at different time points. The parts of the kit of parts can then, e.g., be administered simultaneously or chronologically staggered, that is at different time points and with equal or different time intervals for any part of the kit of parts. The ratio of the total amounts of the combination partner (a) to the combination partner (b) to be administered in the combined preparation can be varied, e.g., in order to cope with the needs of a patient sub-population to be treated or the needs of the single patient based on the severity of any side effects that the patient experiences.

The present invention especially relates to a combined preparation which comprises:
(a) one or more unit dosage forms of a mTOR inhibitor; and
(b) one or more unit dosage forms of an pharmaceutically active agent.

### VII. The Diseases to be Treated

The compositions of the present invention are useful for treating proliferative diseases or diseases that are associated with or triggered by persistent angiogenesis. A proliferative disease is mainly a tumor disease (or cancer) (and/or any metastases). The inventive compositions are particularly useful for treating a tumor which is a breast cancer, genitourinary cancer, lung cancer, gastrointestinal cancer, epidermoid cancer, melanoma, glioma, ovarian cancer, pancreas cancer, neuroblastoma, head and/or neck cancer or bladder cancer, or in a broader sense renal, brain or gastric cancer.

In particular, the inventive compositions are particularly useful for treating:
(i) a breast tumor; a lung tumor, e.g., a small cell or non-small cell lung tumor; melanoma; or
(ii) (ii) a proliferative disease that is refractory to the treatment with other chemotherapeutics; or
(iii) (iii) a tumor that is refractory to treatment with other chemotherapeutics due to multidrug resistance.

Where a tumor, a tumor disease, a carcinoma or a cancer are mentioned, also metastasis in the original organ or tissue and/or in any other location are implied alternatively or in addition, whatever the location of the tumor and/or metastasis.

The compositions are selectively toxic or more toxic to rapidly proliferating cells than to normal cells, particularly in human cancer cells, e.g., cancerous tumors, the compound has significant anti-proliferative effects and promotes differentiation, e.g., cell cycle arrest and apoptosis.

The invention is illustrated by the following Examples.

The following Examples illustrate the combinations with 40-O-(2-hydroxyethyl)-rapamycin that show a syngeristic effect. All combinations were tested in three (3) distinct cell lines as part of this collaboration: A549, a model of non-small cell lung carcinoma; SKOV-3, a model of ovarian cancer; and SKMEL-28, a model of malignant melanoma.

One example is the synergistic effect observed between 40-O-(2-hydroxyethyl)-rapamycin and vinorelbine in A549 cells. In this combination, an increase in the meximum effect was observed compared to either of the single agents alone.

Another example is the synergistic effect observed between 40-O-(2-hydroxyethyl)rapamycin and 8-phenyl-2-(morpholin-4-yl)-chromen-4-one in A549 cells. There was a significant potency shifting of 8-phenyl-2-(morpholin-4-yl)-chromen-4-one by the addition of 40-O-(2-hydroxyethyl)-rapamycin.

All combinations are prepared in the same manner for testing.

### Assay conditions and protocols

### Day 1: Cell preparation

Cells were cultured in T-175 flasks in complete medium (RPMI-1640, 10% FBS, 1% Penn/Strep) at 37 °C and 5% CO2. Cells were removed from the flask by brief treatment with 0.25% trypsin. Trypsin was inactivated with media and cell count was adjusted appropriately. Cells were then seeded into 384-well microtiter plates (35µL) at 1500 (A549) or 3,000 (SKOV-3, SKMEL-28) cells/well using a multi-drop 16-24 hours prior to compound addition for general screening. Seeded plates were incubated (37 °C / 5% CO2) overnight to allow recovery and re-attachment.

### Day 2: Compound addition

Dilution plates were prepared with 100 µL per well of complete medium non-cell culture treated polypropylene 384-well plates. Compounds were added to dilution plates using the Mini-Trak (1 µL addition) for a 1:101 dilution followed by mixing. For single agent dose response curves, a 5µL aliquot from a dilution plate was added to assay plates to generate the 11-point dose responsecurve (final volume 40 µL). Final dilution was ∼1:808 with total solvent concentration ∼0.1%. For combination matrices, 4.5 µL aliquots from dilution plates of orthogonally-titrated master plates were added to the same assay plate to generate the dose-response matrix (final volume of 44 µL). Final dilution of each compound was ∼1:988 with total solvent concentration ∼0.2%. After compound addition, plates were incubated at 37 °C / 5% CO2 for 72 hours.

### Day 5: Measure cell viability

A solution of 5% CellTiter-Blue (Promega) viability dye in complete medium was dispensed to assay plates using a multi-drop or 384-well pipettor. An appropriate volume was added for a final dye concentration of 2.5%. Viability reactions were incubated for 4 to 6 hours depending on cell type at 37 °C / 5% CO2 to allow reduction of viability dye. Plates were allowed to cool to room temperature for one hour before reading fluorescence intensity at 590 nm after excitation at 540 nm in a Wallac Victor-V plate reader.

| **Table III: Cell Lines, Media and Reagents** | | | | |
|---|---|---|---|---|
| | | **Source** | **Catalog #** | **Lot#** |
| **Cell Lines** | | | | |
| | A549 | ATCC | CCL-185 | 3449902 |
| | SKMEL-28 | ATCC | HTB-72 | 348832 |
| | SKOV-3 | ATCC | HTB-77 | 3898710 |

| **Medium and Reagents** | | | | |
|---|---|---|---|---|
| | Base Medium: RPMI-1640^{‡} | ATCC | 30-2001 | |
| | Penicillin/Streptomycin | Cellgro | 30-002-CI | 30002098 |
| | Fetal bovine serum | Gibco | 16000-044 | 1127751 |
| | Trypsin-EDTA (0.25%) | Cellgro | 25-053-CI | 25053103 |
| | L-glutamine | Gibco | 25030-081 | 11150 |
| | Celltiter-Blue Viability Dye | Promega | G8081 | 200719 |

| | | | | |
|---|---|---|---|---|
| ^{‡}Base medium is supplement to create complete medium: 10% FBS, Ponicillin/Streptomycin (1:100), there is no need to add L-glutamine if ATCC medium is used within 3 months after receipt. | | | | |

### QC Criteria

### Primary plate QC status

cHTS plate formats contain groups of positive and negative intra-plate control wells that are used for automated quality control. All assay plates are assigned an automated QC value by the LIM system following data collection. Automatic quality control calls are made based on the Z-factor calculated using intra-plate controls using a standard factor *Z* = 1-3(_V+_U)/(*V*-*U*), where *V,U* are the mean vehicle (treated) and media (untreated) control levels, and _V,_U are the corresponding standard deviation estimates. Z-factor thresholds are empirically set to group plates into three classes: automatically accepted (Z >0.6), automatically rejected (Z <0.4), and undetermined plates that need to be visually evaluated (0.4< Z <0.6). Where necessary the QC status of accepted plates may be reassigned to rejected status based on visual inspection of plate quality, transfer controls or other secondary QC criteria. Plates rejected automatically or by visual inspection are excluded from further analysis and scheduled to be repeated.

### Transfer controls

A positive control compound (Gentian Violet) is included on all master plates. This provides a visual check for screening scientists to verify compound transfer from both column and row masters into the assay plate.

### Secondary QC

Secondary QC includes additional manual checks of data quality including: visual inspection of plate quality and transfer controls, marking of data spikes, and checking for cell-line appropriate behavior of single agents. Plates with an accepted status from primary QC that show an unacceptable plate gradient are adjusted to rejected status and queued for repeat. Plates are also visually inspected for occasional bad wells, or "spikes" with data values that are very different from their immediate neighbors (within the same treatment class). These data spikes are flagged in the database, and excluded from subsequent analyses. Finally, dose-response matrices containing single-agent activity inconsistent with past experience will be marked with rejected status and queued for repeat. Data blocks that did not achieve the cut-off threshold were flagged in the database, excluded from subsequent analysis and queued for repeat as necessary.

### Measuring Antiproliferative Activity

The measure of effect was the inhibition of cell viability using an alamar blue viability assay relative to the untreated level (vehicle alone). For untreated and treated levels U and T, a fractional inhibition I = 1-T/U was calculated. The inhibition ranges from 0% at the untreated level to 100% when T = 0.

Each treated level T was compared to the median untreated level U ± σU, determined for each plate by finding the median alamar blue level (and its associated uncertainty, described above) among the untreated control wells arranged across the plate. Applying standard error propagation rules to the expression for I, the estimated standard error σI ∼ (σU/U) sqrt(1-I).

The error estimates were further increased to account for variations between replicate combination blocks as well as a minimum assumed fractional uncertainty of _min ∼ 3%. Thus for inhibition, the standard error estimate becomes σI ∼ sqrt{ (σU/U)2 (1-I) + σrep 2 + σₘᵢₙ².

### Medians and Error Estimates

Medians were used rather than averages to reduce the effect of occasional outliers on the consensus. While medians are more robust to outliers, they are more sensitive to statistical noise,yielding ∼30% larger deviations. Standard deviations are estimated from the median absolute deviation (MAD), where for a normal distribution, the sample deviation σdat ∼ 1.5 MAD. The standard error for the median itself is then σmed ∼ σdat/sqrt(N-1), given N data values.

### Single Agent Dose Curves

The single agent activity is characterized by fitting a sigmoidal function of the form I = Imax/[1+(C/EC50) ^{σ}], with least squares minimization using a downhill simplex algorithm.Here, C is the concentration, EC50 is the effective concentration at 50% inhibition, and σ is the sigmoidicity. The uncertainty of each fitted parameter was estimated from the range over which the change in reduced chi-squared χ2 is less than one, or less than minimum reduced χ 2 if that minimum exceeds one, to allow for underestimated σI errors. To ensure optimal concentration the EC50 was determined and maximum effect level in each of the proposed proliferation assays. 384-well plates were used , to obtain duplicate dose response curves in 12-step dilutions with a dosing ratio f = 2, 3, or 4, to cover 3-7 orders of magnitude.

### Selecting Optimal Concentrations

We use the single agent curve data to define a dilution series for each compound to be used for combination screening. Using a dilution factor f of 2, 3, or 4, depending on the sigmoidicity of the single agent curve, we will choose 5 dose levels with the central concentration close to the fitted EC50. For compounds with no detectable single agent activity, we will use f = 4 starting from the highest achievable concentration.

### Combination Dose Matrices and Reference Models

The cHTS screening produces dose matrices which contain all pairwise combinations of two single agents at a series of concentrations, including zero. Each dose matrix contains internal copies of the single agent curves which are used as the reference for combination effects. Replicate dose matrices can be merged together by medianing the corresponding data points, and when the concentration series differ, corresponding values are found using bilinear interpolation. Standard errors were computed for each inhibition value using the formulas described above. Combination effects were most readily characterized by comparing each data point's inhibition to that of a combination reference model that was derived from the single agent curves. Three models are generally used: (1) The highest single agent model IHSA(CX,CY) = max(IX,IY) is a simple reference model, where CX,Y are the concentrations of the X and Y compound, and IX,Y are the inhibitions of the single agents at CX,Y; (2) Bliss independence IBliss(CX,CY) = IX + IY - IXIY represents the statistical expectation for independent competing inhibitors; and (3) Loewe additivity, where ILoewe(CX,CY) is the inhibition that satisfies (CX/ECX) + (CY/ECY) = 1, and ECX,Y are the effective concentrations at ILoewe for the single agent curves. Loewe additivity is the generally accepted reference for synergy[4], as it represents the combination response generated if X and Y are the same compound. Both IHSA and IBliss are easily calculated from IX,Y, but determining ILoewe requires interpolation and numerical root finding.

### Selecting Combinations for 9x9 Re-test

To select desirable oncology combinations for repeat assays using high resolution 9x9 dose matrices, three important considerations were evaluated: (1) significant synergy over the additive model; (2) substantial activity where the synergy occurs; and (3) sufficient potency shifting. A "Synergy Score"was used whereby S = log fX log fY _ Idata (Idata-ILoewe), summed over all non-single-agent concentration pairs, and where log fX,Y are the natural logarithm of the dilution factors used for each single agent. This effectively calculates a volume between the measured and Loewe additive response surfaces, weighted towards high inhibition and corrected for varying dilution factors. This volume score emphasizes the overall synergistic or antagonistic effect of the combination, thus minimizing the effects of outlying data spikes and identifying combinations with a robust synergy across a wide range of concentrations and at high effect levels. S is positive for mostly synergistic combinations and negative for antagonism. In cases where both syn rgy and antagonism are present at different concentrations, the weighting favors effects at high inhibition levels. An uncertainty σS is calculated for each synergy score, based on the measured errors for the Idata values and standard error propagation. The synergy score was used and its error to define an appropriate selection cutoff. For example, combinations with S > 2_S are significant at ∼95% confidence, assuming a normal distribution. Also, to ensure a sufficient potency shift, the combination index, CI = (CX/ECX) + (CY/ECY) at a chosen effect level is small enough to represent a useful synergy. Observed *in vitro* CI measurements for currently used clinical combinations (CI ∼ 0.5-0.7) can be used as a guide in setting the cutoff.

The Table below lists the combinations showing the best synergy with 40-O-(2-hydroxyethyl)-rapamycin

| Combination | Synergy Score | Cell Line |
|---|---|---|
| 40-O-(2-hydroxyethyl)-rapamycin + *N*-[1-cyclohexyl-2-oxo-2-(6-phenethyl-octahydro-pyrrolo[2,3-*c*]pyridin-1-yl-ethyl]-2-methylamino-propionamide | 2.550 | SKOV3 |
| 40-O-(2-hydroxyethyl)-rapamycin + N1 N12-diethylspermine 4HCl | 0.901 | SKMEL28 |
| 40-O-(2-hydroxyethyl)-rapamycin + 8-phenyl-2-(morpholin-4-yl)-chromen-4-one | 1.217 | SKMEL28 |
| 40-O-(2-hydroxyethyl)-rapamycin + Thiotepa | 0.779 | A549 |
| 40-O-(2-hydroxyethyl)-rapamycin + Carboplatin + vinorelbine | 1.475 | A549 |
| 40-O-(2-hydroxyethyl)-rapamycin + vindesine sulfate | 1.315 | A549 |
| 40-O-(2-hydroxyethyl)-rapamycin + mitoxantrone hydrochloride +prednisone | 0.996 | SKMEL28 and A549 |
| 40-O-(2-hydroxyethyl-)rapamycin + 1 H-pyrazolo-[3,4-d]pyrimidin-4-amine, 3-(4-chlorophenyl)-1-(1,1-dimethylethyl)-(9Cl) | 0.974 | A549 |
| 40-O-(2-hydroxyethyl)-rapamycin + Idarubicin hydrochloride | 0.953 | A549 |
| 40-O-(2-hydroxyethyl)-rapamycin + cladribine | 0.872 | SKMEL28 and A549 |
| 40-O-(2-hydroxyethyl)-rapamycin + cytarabine | 0.940 | A549 |
| 10-Hydroxycamptothecin | 1.268 | A549 |
| Carboplatin + Docetaxel | 1.054 | A549 |
| Vinorelbine | 0.887 | SKOV3 A549 |
| Vincristine Sulfate | 2.351 | SKME28 |
| Staurosporine | 0.983 | SKOV3 |

## Claims

1. A combination of
(a) a mTOR inhibitor compound; and
(b) one or more pharmaceutically active agents selected from the group consisting of:
i. a microtubule binding agent;
ii. an EGFR, PDGFR tyrosine kinase inhibitor;
iii. an adjuvant;
iv. an adrenal cortex antagonist;
v. AKT pathway inhibitor;
vi. An alkylating agent;
vii. an angiogenesis inhibitor;
viii. an anti-androgen;
ix. an anti-estrogen;
x. an anti-hypercalcemia agent;
xi. an antimetabolite;
xii. an apoptosis inducer;
xiii. an aurora kinase inhibitor;
xiv. a Bruton's Tyrosine Kinase (BTK) inhibitor;
xv. a calcineurin inhibitor;
xvi. a CaM kinase II inhibitor;
xvii. a CD45 tyrosine phosphatase inhibitor;
xviii.a CDC25 phosphatase inhibitor;
xix. a CHK kinase inhibitor;
xx. a controlling agent for regulating genistein, olomucine and/or tyrphostins;
xxi. a cyclooxygenase inhibitor;
xxii. a cRAF kinase inhibitor;
xxiii.a cyclin dependent kinase inhibitor;
xxiv. a cysteine protease inhibitor;
xxv. a DNA intercalator;
xxvi. a DNA strand breaker;
xxvii. an E3 Ligase inhibitor;
xxviii. an endocrine hormone;
xxix. compounds targeting, decreasing or inhibiting the activity of the epidermal growth factor family;
xxx. an EGFR, PDGFR tyrosine kinase inhibitor;
xxxi. a farnesyltransferase inhibitor;
xxxii. a Flk-1 kinase inhibitor;
xxxiii. a Glycogen synthase kinase-3 (GSK3) inhibitor;
xxxiv. a histone deacetylase (HDAC) inhibitor;
xxxv. a HSP90 inhibitor;
xxxvi. a I-kappa B-alpha kinase inhibitor (IKK);
xxxvii. an insulin receptor tyrosine kinase inhibitor;
xxxviii. a c-Jun N-terminal kinase (JNK) kinase inhibitor;
xxxix. a microtubule binding agent;
xl. a Mitogen-activated protein (MAP) kinase-inhibitor;
xli.a MDM2 inhibitor;
xlii. a MEK inhibitor;
xliii. a matrix metalloproteinase inhibitor (MMP) inhibitor;
xliv. a NGFR tyrosine-kinase-inhibitor;
xlv. a p38 MAP kinase inhibitor, including a SAPK2/p38 kinase inhibitor;
xlvi. a p56 tyrosine kinase inhibitor;
xlvii.a PDGFR tyrosine kinase inhibitor;
xlviii. a phosphatidylinositol 3-kinase inhibitor;
xlix. a phosphatase inhibitor;
l. a platinum agent;
li. a protein phosphatase inhibitor, including a PP1 and PP2 inhibitor and a tyrosine phosphatase inhibitor;
lii. a PKC inhibitor and a PKC delta kinase inhibitor;
liii. a polyamine synthesis inhibitor;
liv. a proteosome inhibitor;
lv. a PTP1B inhibitor;
lvi. a protein tyrosine kinase inhibitor including a SRC family tyrosine kinase inhibitor; a Syk tyrosine kinase inhibitor; and a JAK-2 and/or JAK-3 tyrosine kinase inhibitor;
lvii. a retinoid;
lviii. a RNA polymerase II elongation inhibitor;
lix. a serine/threonine kinase inhibitor;
lx. a sterol biosynthesis inhibitor;
lxi. a topoisomerase inhibitor;
lxii. VEGFR tyrosine kinase inhibitor.
lxiii. a gonadorelin agonist,
lxiv. a compound which induce cell differentiation processes,
lxv. a bisphosphonate,
lxvi. a heparanase inhibitor,
lxvii.a biological response modifier,
lxviii. a telomerase inhibitor,
lxix. inhibitors of catechol-O-methyltransferase,
lxx. an immunosuppressive monoclonal antibody to leukocyte receptors,
lxxi. somatostatin or a somatostatin analogue,
lxxii.Growth Hormone-Receptor Antagonists,:
lxxiii. monoclonal antibodies useful for leukemia (AML) treatment,
lxxiv. antibodies against carcinoembryonic antigen (CEA),
lxxv. phosphodiesterase inhibitor,
lxxvi. cancer vaccine,
lxxvii. inhibitors of Kinesin Spindle Protein (KSP),
lxxviii. inhibitors of multiple receptor tyrosine kinases associated with tumour growth and angiogenesis,
lxxix. synthetic nonsteroidal estrogens,
lxxx. cytoxic antineoplastics,
lxxxi. a recombinant binding molecule having at least a portion of the extracellular domain of CTLA4 or a mutant thereof.
for simultaneous, concurrent, separate or sequential use in for preventing or treating a proliferative disease.

2. A combination of claim 1, wherein the one or more pharmaceutically active agents are selected from i. to xii.

3. The combination according toany one of claim 1 or 2, wherein the one or more pharmaceutically active agents are selected from the group consisting of a microtubule binding agent or an EGFR, PDGFR tyrosine kinase inhibitor, a steroid, an alkylating agent; an antimetabolite; phosphatidylinositol 3-kinase inhibitor; a platinum agent; a polyamine synthesis inhibitor; a SRC family tyrosine kinase inhibitor; a topoisomerase inhibitor; or a mixture thereof.

4. A combination according to clam 1, wherein one or more pharmaceutically active agents are selected from the group consisting of prednisone, Thiotepa; N1, N12-diethylspermine 4HCl , 8-phenyl-2-(morpholin-4-yl)-chromen-4-one, 1 H-pyrazolo-[3,4-d] pyrimidin-4-amine, 3-(4-chlorophenyl)-1-(1,1-dimethylethyl)-(9Cl), Cytarabine; Cladribine; Vindesine sulfate; Vinorelbine; Carboplatin; idarubicin hydrochloride; and mitoxantrone hydrochloride, and a mixture thereof.

5. A pharmaceutical composition comprising a combination of any one of claims 1 to 4.

6. A commercial package comprising a combination of any one of claims 1 to 4.

7. A commercial package of claim 6, wherein the unit dosage form is a fixed combination.

8. The use of a combination, pharmaceutical composition or commercial package according to any one of claims 1 to 7, for the manufacture of a medication for preventing or treating proliferative diseases or diseases that are associated with or triggered by persistent angiogenesis in a mammal.

9. A method of preventing or treating a proliferative disease comprising the combination of any one of claims 1 to 4, a pharmaceutical composition according to claim 5, or a pharmaceutical package according to any one of claims 6 or 7.

10. A combination, a pharmaceutical composition, a commercial package, the use or a method according to any one of claims 1 to 9, wherein the proliferative disease is selected from ovarian cancer, lung carcinoma and melanoma.

11. A combination, a pharmaceutical composition, a commercial package, the use or a method according to any preceding claim, wherein the mTOR inhibitor compound is selected from the group consisting of rapamycin, 40-O-alkyl-rapamycin derivatives, 40-O-hydroxyalkyl-rapamycin derivatives, 40-O-alkoxyalkyl-rapamycin derivatives, 32-deoxo-rapamycin and 32-hydroxy-rapamycin derivatives, 16-O-substituted rapamycin derivatives, rapamycin derivatives which are acylated at the oxygen group in position 40, rapamycin derivatives which are substituted in position 40 by heterocyclyl and 40-O-phospho-containing rapamycin derivatives.

12. A combination, a pharmaceutical composition, a commercial package, the use or a method according to any preceding claim, wherein the mTOR inhibitor compound is selected from the group consisting of 40-O-(2-hydroxy)-ethyl-rapamycin, CCI779, ABT578, or AP23573,

13. A combination, a pharmaceutical composition, a commercial package, the use or a method according to any preceding claim, wherein the mTOR inhibitor compound is 40-O-(2-hydroxy)-ethyl-rapamycin.

14. A combination, a pharmaceutical composition, a commercial package, the use or a method according to any one of claims 1 to 11, wherein the mTOR inhibitor compound is a compound of formula 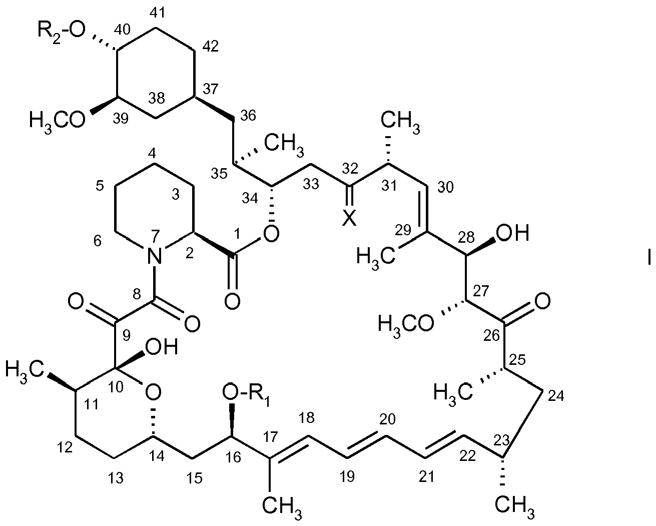 wherein
R₁ is CH₃ or C₃₋₆alkynyl,
R₂ is H or -CH₂-CH₂-OH, and
X is =O, (H,H) or (H,OH)
provided that R₂ is other than H when X is =O and R₁ is CH₃.
